# EUROPEAN PATENT SPECIFICATION

(11) **EP 3 930 826 B1**
(45) Date of publication and mention of the grant of the patent: **25.02.2026**
(21) Application number: 20763704.2
(22) Date of filing: 20.02.2020
(51) Int. Cl.: A61N 1/36, A61N 1/08, A61N 1/05, H04R 25/00

(54) **PROSTHESIS OPERATION IN THE FACE OF MAGNETIC FIELDS**
PROTHESENBETRIEB ANGESICHTS VON MAGNETFELDERN
OPÉRATION DE PROTHÈSE FACE À DES CHAMPS MAGNÉTIQUES

(30) Priority: 26.02.2019 US 201962810884 P
(43) Date of publication of application: 05.01.2022
(73) Proprietor: Cochlear Limited, Macquarie University, New South Wales 2109 (AU)
(72) Inventor: FIERENS, Guy, Macquarie University, New South Wales 2109 (AU); VERMA, Rishubh, Macquarie University, New South Wales 2109 (AU); RAMBAULT, Antonin, Macquarie University, New South Wales 2109 (AU); MEEUSEN, Tom, Macquarie University, New South Wales 2109 (AU); LEROUX, Thomas, Macquarie University, New South Wales 2109 (AU)
(74) Representative: Grünecker Patent- und Rechtsanwälte PartG mbB
(86) International application number: PCT/IB2020/051448
(87) International publication number: WO 2020/174330

(56) References cited:
- WO-A1-2006/058368
- WO-A1-2014/158191
- WO-A1-2015/132692
- US-A1- 2005 001 703
- US-A1- 2007 167 671
- US-A1- 2009 163 980
- US-A1- 2009 163 981
- US-A1- 2010 198 312
- US-A1- 2012 219 166
- US-A1- 2014 100 637
- US-A1- 2014 100 637
- US-A1- 2018 353 761
- US-B1- 6 496 734
- US-B2- 9 174 058

## Description

### BACKGROUND

Hearing loss is generally of two types, conductive and sensorineural. Sensorineural hearing loss is due to the absence or destruction of the cochlear hair cells which transduce sound into nerve impulses. Various hearing prostheses have been developed to provide individuals suffering from sensorineural hearing loss with the ability to perceive sound. For example, cochlear implants have an electrode assembly which is implanted in the cochlea. In operation, electrical stimuli are delivered to the auditory nerve via the electrode assembly, thereby bypassing the inoperative hair cells to cause a hearing percept.

Conductive hearing loss occurs when the natural mechanical pathways that provide sound in the form of mechanical energy to cochlea are impeded, for example, by damage to the ossicular chain or ear canal. For a variety of reasons, such individuals are typically not candidates for a cochlear implant. Rather, individuals suffering from conductive hearing loss typically receive an acoustic hearing aid. Hearing aids rely on principles of air conduction to transmit acoustic signals to the cochlea. In particular, hearing aids amplify received sound and transmit the amplified sound into the ear canal. This amplified sound reaches the cochlea in the form of mechanical energy, causing motion of the perilymph and stimulation of the auditory nerve.

Not all individuals suffering from conductive hearing loss are able to derive suitable benefit from hearing aids. For example, some individuals are prone to chronic inflammation or infection of the ear canal. Other individuals have malformed or absent outer ear and/or ear canals resulting from various scenarios. For these and other individuals, another type of hearing prosthesis has been developed in recent years. This hearing prosthesis, commonly referred to as a middle ear implant, converts received sound into a mechanical force that is applied to the ossicular chain or directly to the cochlea via an actuator implanted in or adjacent to the middle ear cavity. Conversely, cochlear implants can have utilitarian value
with respect to recipients where all of the inner hair inside the cochlea has been damaged or otherwise destroyed. Electrical impulses are provided to electrodes located inside the cochlea, which stimulate nerves of the recipient so as to evoke a hearing percept.
US 2005/001703 A1 and US2012219166 A1 relate to an electromagnetic driver transducer free of torque in the presence of an external magnetic field, of any direction and orientation (such as in a Magnetic Resonance Imaging environment). US 2009/163981 A1 relates to an RI-compatible electronic medical therapy system that includes an active medical device connected to a plurality of electrodes. A multiplexer circuit includes at least one circuit protection device in electrical series with the electrodes and the medical device. The circuit protection device can be in the form of a switch or a band stop filter. The circuit protection device is adapted to permit current flow therethrough during normal medical device related therapy, but substantially prevent current flow therethrough in the presence of an induced electromagnetic field.

### SUMMARY

The invention is defined by the claims.

In accordance with an exemplary embodiment, there is an apparatus, comprising an implantable portion of a hearing prosthesis, wherein the apparatus is configured to at least partially cancel a signal in the implantable portion, the signal resulting from an external magnetic field generated external to a recipient of the hearing prosthesis.

In accordance with another exemplary embodiment, there is an apparatus, comprising an implantable hearing prosthesis, wherein the implantable hearing prosthesis is configured to function to reduce and/or eliminate a hearing percept that would be evoked by the hearing prosthesis when subjected to an external magnetic field generated external to a recipient of the hearing prosthesis in the absence of the functioning.

In accordance with another exemplary embodiment, there is an apparatus, comprising an implantable prosthesis, wherein the implantable prosthesis is configured to provide a signal to an actuator of the prosthesis to actuate the actuator at a different frequency and/or amplitude relative to a phenomenon induced signal induced in the prosthesis that otherwise results in a respective actuation frequency and/or amplitude induced by a phenomenon unrelated to the prosthesis while the phenomenon is present, wherein the signal to actuate the actuator at the different frequency and/or amplitude at least partially mitigates the effects of the phenomenon induced signal.

In accordance with another exemplary embodiment, there is a method, comprising exposing a recipient of a prosthesis to an MRI field, wherein the exposure also results in exposure of the prosthesis to the field and operating the prosthesis to operate in a different manner than that which is normally the case while the recipient is exposed to the MRI field.

### BRIEF DESCRIPTION OF THE DRAWINGS

Some embodiments are described below with reference to the attached drawings, in which:
FIG. l is a perspective view of an exemplary bone conduction device in which at least some embodiments can be implemented;
FIGs. 2A and 2B are schematic diagrams illustrating exemplary middle ear implants according to some exemplary embodiments;
FIG. 3 depicts an exemplary embodiment of an implanted tube microphone utilized in conjunction with a cochlear implant;
FIGs. 4A and 4B illustrate some exemplary embodiments of the exemplary tube microphones;
FIG. 5 illustrates an exemplary embodiment of an implantable apparatus;
FIGs. 6-11 present exemplary functional schematics of some exemplary embodiments;
FIGs. 12 and 13 present exemplary schematics of an electromechanical actuator; and
FIG. 14 presents an exemplary flowchart for an exemplary method.

### DETAILED DESCRIPTION

Aspects of the present invention are generally directed to an implantable component of a middle ear hearing prosthesis. A middle ear transducer is operationally coupled to the receiver-stimulator, and a transducer fixation mechanism is connected to (in some embodiments, is an integral part of) the transducer, and extends from the transducer into the middle ear cavity. While some of the embodiments detailed herein are directed towards hearing prostheses in general and middle ear implants (direct acoustic cochlear stimulator, as such is sometimes referred to) in particular, other embodiments include an implanted transducer used for sensing sound (implanted microphone, for example) or sensing other phenomenon. Moreover, while the teachings detailed herein are sometimes directed towards a transducer in the form of an actuator, any such disclosure corresponds to a disclosure in an alternate embodiment of a transducer in the form of a microphone (and *vice versa*). Also, while the embodiments detailed herein are directed towards an embodiment where the prosthesis is a middle ear hearing prosthesis, in other embodiments, the teachings detailed herein, can be applicable to other technologies, such as a cochlear implant that is a totally implantable hearing prosthesis with an implanted transducer, or any other technology that has an implanted transducer where the magnetic field effects detailed herein can result in one or more of the phenomena detailed herein or variations thereof or other phenomena (e.g., a mechanical actuator implanted in a cochlea that imparts mechanical force to tissue inside the cochlea, an optical stimulator implanted in the cochlea). Embodiments of the teachings herein include active bone conduction devices implanted in a recipient (e.g., where the actuator herein is configured to vibrate skull bone, such as the mastoid bone (the actuator can be directly attached to a surface thereof) to evoke a hearing percept). Embodiments of the teachings herein include devices that utilize actuators to move tissue or impart energy into the recipient beyond hearing prosthesis related devices. For example, a heart actuator or a penile implant or an artificial limb actuator or a jaw actuator. The teachings herein can be directed to body fluid pumps that are implanted in the recipient. Moreover, embodiments of the teachings herein can include components that are not implanted in a recipient and may not even be medical devices in the first instance, but are devices that would be exposed to a magnetic field or an electromagnetic field. Also, while implanted transducers herein are directed to sound sensors, these devices may not be implanted. Also, the transducers may not necessarily be sound sensors, but sensors that sense other phenomenon, such as muscle movement, blood pressure, temperature (a mechanical sensor could be used to sense such), etc. Any transducer to which the teachings herein can be applied can be used in some embodiments. The teachings herein can be applicable to bionic eyes / retinal implants, etc. Accordingly, embodiments can include any apparatus disclosed herein in combination with the specific magnetic field / EM field management related teachings herein. Embodiments can include any apparatus disclosed herein in combination with the specific magnetic field / EM field management related teachings herein to counter at least in part (including eliminate) the sensory effects from the apparatus being exposed to the aforementioned fields.

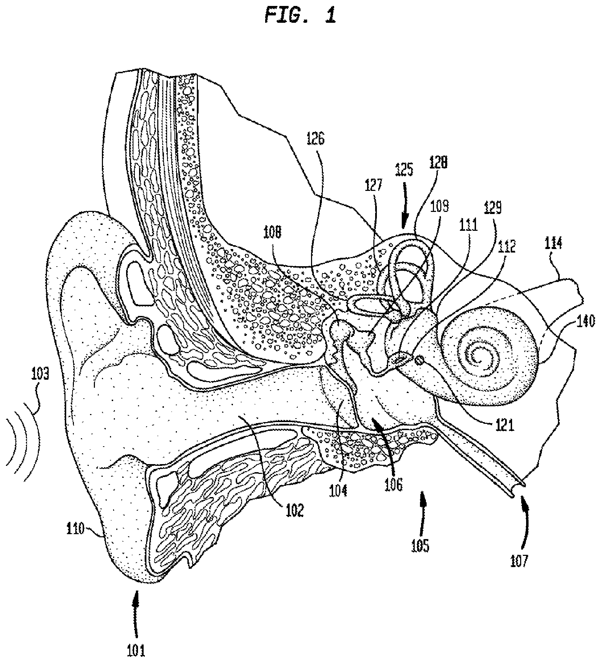

FIG. 1 is a perspective view of a human skull showing the anatomy of the human ear. As shown in FIG. 1, the human ear comprises an outer ear 101, a middle ear 105, and an inner ear 107. In a fully functional ear, outer ear 101 comprises an auricle 110 and an ear canal 102. An acoustic pressure or sound wave 103 is collected by auricle 110 and channeled into and through ear canal 102. Disposed across the distal end of ear canal 102 is a tympanic membrane 104 which vibrates in response to sound wave 103. This vibration is coupled to oval window or fenestra ovalis 112, which is adjacent round window 121. This vibration is coupled through three bones of middle ear 105, collectively referred to as the ossicles 106 and comprising the malleus 108, the incus 109, and the stapes 111. Bones 108, 109, and 111 of middle ear 105 serve to filter and amplify sound wave 103, causing oval window 112 to articulate, or vibrate in response to the vibration of tympanic membrane 104. This vibration sets up waves of fluid motion of the perilymph within cochlea 140. Such fluid motion, in turn, activates hair cells (not shown) inside cochlea 140. Activation of the hair cells causes nerve impulses to be generated and transferred through the spiral ganglion cells (not shown) and auditory nerve 114 to the brain (also not shown) where they cause a hearing percept.

As shown in FIG. 1, semicircular canals 125 are three half-circular, interconnected tubes located adjacent cochlea 140. Vestibule 129 provides fluid communication between semicircular canals 125 and cochlea 140. The three canals are the horizontal semicircular canal 126, the posterior semicircular canal 127, and the superior semicircular canal 128. The canals 126, 127, and 128 are aligned approximately orthogonally to one another. Specifically, horizontal canal 126 is aligned roughly horizontally in the head, while the superior 128 and posterior canals 127 are aligned roughly at a 45-degree angle to a vertical through the center of the individual's head.

Each canal is filled with a fluid called endolymph and contains a motion sensor with tiny hairs (not shown) whose ends are embedded in a gelatinous structure called the cupula (also not shown). As the orientation of the skull changes, the endolymph is forced into different sections of the canals. The hairs detect when the endolymph passes thereby, and a signal is then sent to the brain. Using these hair cells, horizontal canal 126 detects horizontal head movements, while the superior 128 and posterior 127 canals detect vertical head movements.

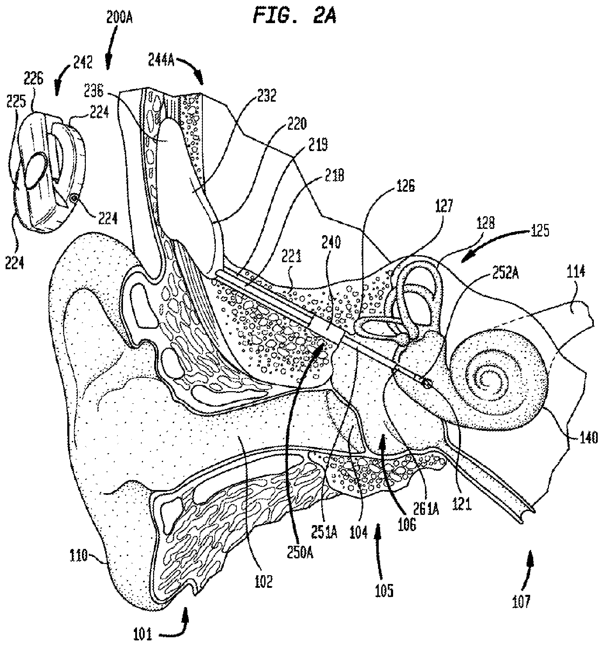

FIG. 2A is a perspective view of an exemplary direct acoustic cochlear stimulator 200A in accordance with embodiments of the present invention. (Sometimes herein, this is referred to as a middle ear implant.) Direct acoustic cochlear stimulator 200A comprises an external component 242 that is directly or indirectly attached to the body of the recipient, and an internal component 244A that is temporarily or permanently implanted in the recipient. External component 242 typically comprises two or more sound input elements, such as microphones 224, for detecting sound, a sound processing unit 226, a power source (not shown), and an external transmitter unit 225. External transmitter unit 225 comprises an external coil (not shown). Sound processing unit 226 processes the output of microphones 224 and generates encoded data signals which are provided to external transmitter unit 225. For ease of illustration, sound processing unit 226 is shown detached from the recipient. Internal component 244A comprises an internal receiver unit 232, a stimulator unit 220, and a stimulation arrangement 250A in electrical communication with stimulator unit 220 via cable 218 extending through artificial passageway 219 in mastoid bone 221. Internal receiver unit 232 and stimulator unit 220 are hermetically sealed within a biocompatible housing, and are sometimes collectively referred to as a stimulator/receiver unit.

Internal receiver unit 232 comprises an internal coil (not shown), and optionally, a magnet (also not shown) fixed relative to the internal coil. The external coil transmits electrical signals (i.e., power and stimulation data) to the internal coil via a radio frequency (RF) link. The internal coil is typically a wire antenna coil comprised of multiple turns of electrically insulated platinum or gold wire. The electrical insulation of the internal coil is provided by a flexible silicone molding (not shown). In use, implantable receiver unit 232 is positioned in a recess of the temporal bone adjacent auricle 110.

In the illustrative embodiment of FIG. 2A, ossicles 106 have been explanted. However, it should be appreciated that stimulation arrangement 250A may be implanted without disturbing ossicles 106.

Stimulation arrangement 250A comprises an actuator 240, a stapes prosthesis 252A and a coupling element 251A which includes an artificial incus 261B. Actuator 240 is osseointegrated to mastoid bone 221, or more particularly, to the interior of artificial passageway 219 formed in mastoid bone 221.

In this embodiment, stimulation arrangement 250A is implanted and/or configured such that a portion of stapes prosthesis 252A abuts an opening in one of the semicircular canals 125. For example, in the illustrative embodiment, stapes prosthesis 252A abuts an opening in horizontal semicircular canal 126. In alternative embodiments, stimulation arrangement 250A is implanted such that stapes prosthesis 252A abuts an opening in posterior semicircular canal 127 or superior semicircular canal 128.

As noted above, a sound signal is received by microphone(s) 224, processed by sound processing unit 226, and transmitted as encoded data signals to internal receiver 232. Based on these received signals, stimulator unit 220 generates drive signals which cause actuation of actuator 240. The mechanical motion of actuator 240 is transferred to stapes prosthesis 252A such that a wave of fluid motion is generated in horizontal semicircular canal 126. Because vestibule 129 provides fluid communication between the semicircular canals 125 and the median canal, the wave of fluid motion continues into the median canal, thereby activating the hair cells of the organ of Corti. Activation of the hair cells causes appropriate nerve impulses to be generated and transferred through the spiral ganglion cells (not shown) and auditory nerve 114 to cause a hearing percept in the brain.

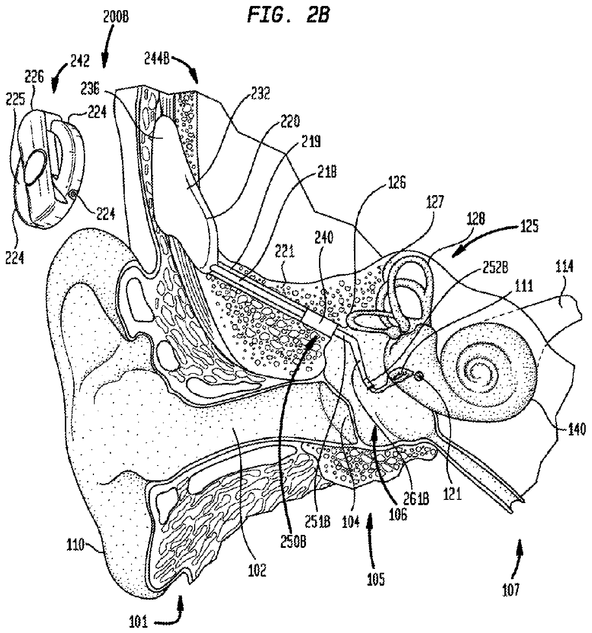

FIG. 2B depicts an exemplary embodiment of a middle ear implant 200B having a stimulation arrangement 250B comprising actuator 240 and a coupling element 251B. Coupling element 251B includes a stapes prosthesis 252B and an artificial incus 261B which couples the actuator to the stapes prosthesis. In this embodiment, stapes prosthesis 252C abuts stapes 111.

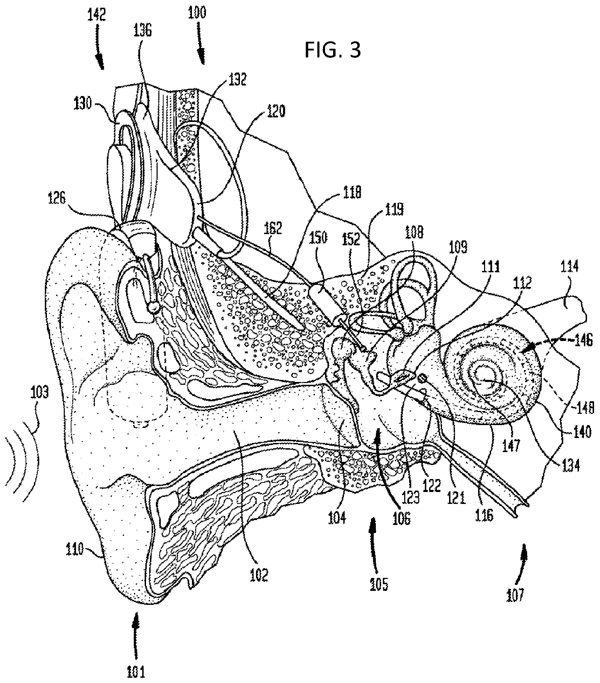

FIG. 3 is perspective view of a totally implantable cochlear implant, referred to as cochlear implant 100, implanted in a recipient. As shown, cochlear implant 100 comprises one or more components which are temporarily or permanently implanted in the recipient. Cochlear implant 100 is shown in FIG. 3 with an external device 142 which, as described below, is configured to provide power to the cochlear implant.

In the illustrative arrangement of FIG. 3, external device 142 may comprise a power source (not shown) disposed in a Behind-The-Ear (BTE) unit 126. External device 142 also includes components of a transcutaneous energy transfer link, referred to as an external energy transfer assembly. The transcutaneous energy transfer link is used to transfer power and/or data to cochlear implant 100. As would be appreciated, various types of energy transfer, such as infrared (IR), electromagnetic, capacitive and inductive transfer, may be used to transfer the power and/or data from external device 142 to cochlear implant 100. In the illustrative embodiments of FIG. 1, the external energy transfer assembly comprises an external coil 130 that forms part of an inductive radio frequency (RF) communication link. External coil 130 is typically a wire antenna coil comprised of multiple turns of electrically insulated single-strand or multi-strand platinum or gold wire. External device 142 also includes a magnet (not shown) positioned within the turns of wire of external coil 130. It should be appreciated that the external device shown in FIG. 3 is merely illustrative, and other external devices may be used with embodiments of the present invention.

Cochlear implant 100 comprises an internal energy transfer assembly 132 which may be positioned in a recess of the temporal bone adjacent auricle 110 of the recipient. As detailed below, internal energy transfer assembly 132 is a component of the transcutaneous energy transfer link and receives power and/or data from external device 142. In the illustrative embodiment, the energy transfer link comprises an inductive RF link, and internal energy transfer assembly 132 comprises a primary internal coil 136. Internal coil 136 is typically a wire antenna coil comprised of multiple turns of electrically insulated single-strand or multi-strand platinum or gold wire.

Cochlear implant 100 further comprises a main implantable component 120 and an elongate electrode assembly 118. In embodiments of the present invention, internal energy transfer assembly 132 and main implantable component 120 are hermetically sealed within a biocompatible housing. In embodiments of the present invention, main implantable component 120 includes a sound processing unit (not shown) to convert the sound signals received by the implantable microphone in internal energy transfer assembly 132 to data signals. Main implantable component 120 further includes a stimulator unit (also not shown) which generates electrical stimulation signals based on the data signals. The electrical stimulation signals are delivered to the recipient via elongate electrode assembly 118.

Elongate electrode assembly 118 has a proximal end connected to main implantable component 120, and a distal end implanted in cochlea 140. Electrode assembly 118 extends from main implantable component 120 to cochlea 140 through mastoid bone 119. In some embodiments, electrode assembly 118 may be implanted at least in basal region 116, and sometimes further. For example, electrode assembly 118 may extend towards apical end of cochlea 140, referred to as cochlea apex 134. In certain circumstances, electrode assembly 118 may be inserted into cochlea 140 via a cochleostomy 122. In other circumstances, a cochleostomy may be formed through round window 121, oval window 112, the promontory 123 or through an apical turn 147 of cochlea 140.

Electrode assembly 118 comprises a longitudinally aligned and distally extending array 146 of electrodes 148, sometimes referred to as electrode array 146 herein, disposed along a length thereof. Although electrode array 146 may be disposed on electrode assembly 118, in most practical applications, electrode array 146 is integrated into electrode assembly 118. As such, electrode array 146 is referred to herein as being disposed in electrode assembly 118. As noted, a stimulator unit generates stimulation signals which are applied by electrodes 148 to cochlea 140, thereby stimulating auditory nerve 114.

As noted, cochlear implant 100 comprises a totally implantable prosthesis that is capable of operating, at least for a period of time, without the need for external device 142. Therefore, cochlear implant 100 further comprises a rechargeable power source (not shown) that stores power received from external device 142. The power source may comprise, for example, a rechargeable battery. During operation of cochlear implant 100, the power stored by the power source is distributed to the various other implanted components as needed. The power source may be located in main implantable component 120, or disposed in a separate implanted location.

Stimulator 132 receives a signal generated by an implanted sound sensor 150, in this embodiment, via a cable 162. Sound sensor 150 is implanted in a cavity formed in mastoid bone 119 so as to extend, in this embodiment, into the middle ear cavity. Sound sensor 150 is configured to detect sound received in a recipient's ear through the use of vibrations or pressure variations that occur in or along the natural path that is followed by acoustic waves in the ear. More specifically, sound sensor 150 senses vibration of a structure of the recipient's ear or vibration of fluid within one of the recipient's body cavities, such as the recipient's middle ear cavity, inner ear canals, cochlear ducts, etc. The vibration of the recipient's ear structure, or the vibration of the fluid within a body cavity is a result of the receipt of acoustic waves that travel from the recipient's outer ear to the middle and inner ear. That is, the received acoustic waves result in the vibration of the middle or inner ear structures, or travel through the middle ear cavity, creating vibration of the fluid within the cavities. In the embodiment illustrated in FIG. 3, the sound sensor detects sound based on vibration of the recipient's middle ear bones, and more specifically, based on vibration of incus 109. In an exemplary embodiment, sound sensor is an electro-magnetic transducer, and in other embodiments, it is a piezo-electric transducer. In some embodiments, one or more of the features detailed below with respect to the actuator below are applicable to the sound sensor 150, and are present therewith. Corollary to this is that any one or more of the teachings associated with the microphones herein can be present in the actuators disclosed herein.

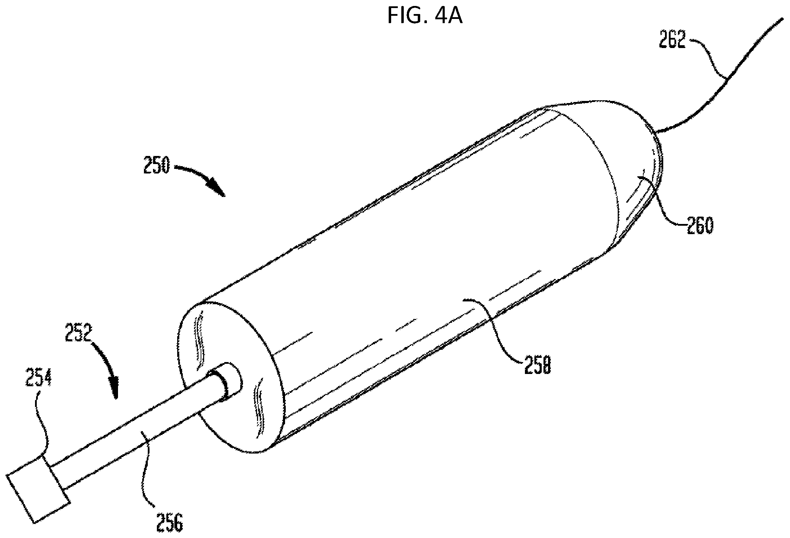

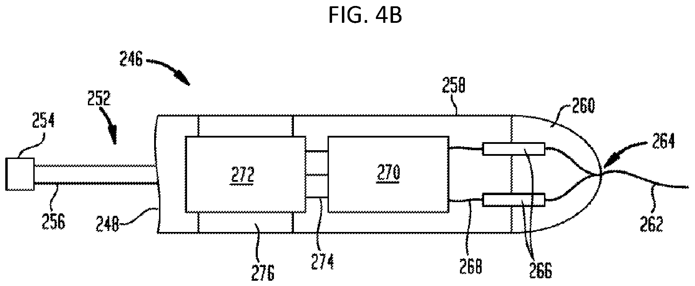

An embodiment of implantable sound sensor 150 is described next below with reference to FIGS. 4A and 4B, referred to herein as implantable sound sensor 250. Implantable sound sensor 250 comprises a housing 258 having, in this embodiment, a substantially tubular shape. The tubular shape may have a cylindrical or elliptical cross-sectional shape. Other shapes, such as prismatic with square, rectangular, or other polygonal cross-sectional shapes may also be used in alternative embodiments. However, a cylindrical shape may be advantageous for purposes of implantation and manufacture.

In the embodiments of FIGS. 4A and 4B, housing 258 is closed at one end 246 by a membrane 248. Membrane 248 is connected to housing 258 as to hermetically seal the one end 246. Membrane 248 may be connected to housing 258 through one of many known techniques, such as laser welding or manufacturing (milling, turning) housing 258 and membrane 248 out of one piece.

Housing 258 is closed at the opposing end 264, that is, the end remote from membrane 248, by a closure 260. Closure 260 also provides a hermetical seal. Hence, housing 258, membrane 248 and closure 260 form a biocompatible hermetically-sealed enclosure that is substantially impenetrable to air and body fluids.

In embodiments of the present invention, membrane 248 is substantially flexible and is configured to vibrate. The thickness of membrane 248 is selected depending on, for example, the material of which it is made and the body location in which sound sensor 250 will be implanted. Additionally, membrane 248 and housing 258 may be each made from the same or different titanium or a titanium alloy. However, it would be appreciated that other biocompatible materials may also be used. For example, in one alternative embodiment, closure 260 may be manufactured of a biocompatible ceramic material.

A coupling mechanism 252 is secured to the exterior surface of membrane 248. In the embodiment illustrated in FIGs. 4A and 4B, coupling mechanism 252 comprises an elongate rod 256 and a bracket 254 disposed on the distal end of the rod. Bracket 254 may have a variety of configurations depending on which structure of the natural ear the device is to be secured. This is described in further detail below.

A vibrational sensor 272, such as a microphone, is disposed inside housing 258. In certain such embodiments, the vibrational sensor is a pressure sensitive transducer configured to generate an electrical signal in response to detected pressure waves. Microphone 272 may be arranged such that the microphone's sensing element is located proximal to membrane 248 with a defined gas layer 275 positioned between the microphone's sensing element and the membrane. The microphone's sensing element is typically a diaphragm.

The housing 258 is a cylindrical component, as can be seen. In an exemplary embodiment, the microphone 250 has any one or more or all of the features of the transducer disclosed in U.S. Patent Application No. 12/997,788, entitled Implantable Sound Sensor for Hearing Prostheses, to Koen Erik Van den Heuvel.

In the above embodiments, as coupling mechanism 252 vibrates membrane 248, the excitation of the membrane is transmitted to the inside of housing 258, where it is sensed by microphone 272. Microphone 272 may be an electret microphone, such as from Sonion (Denmark) or Knowles (USA). Other types of microphones may be used as well, such as: magnetic, dynamic, piezo-electric, optical, or electro-mechanical.

In an alternative embodiment, vibrational sensor 272 is an accelerometer suitable for sensing vibrations of membrane 248. In one particular embodiment, vibrational sensor 272 is a micro-electromechanical system accelerometer.

Vibrational sensor 272 is connected to housing 258 by means of a fluid suspension 276, which is preferably made of, or that comprises, silicone. It should be appreciated that in alternative embodiments, other mechanisms may be implemented to isolate vibrational sensor 272 from the motion of sound sensor 250.

Implantable sound sensor 250 further comprises a transmitter for transmitting the signal generated by vibrational sensor 272, either raw or processed, to an element outside of sound sensor 250, such as to an implantable stimulation device or other component of an implantable hearing prosthesis.

The transmitter may comprise an electronic circuit 270 mounted inside housing 258 that is coupled to microphone 272 by wires 262. Electronic circuit 270 may be configured to process the signal generated by the microphone 272 for transmission to an implantable stimulation device.

Electronic circuit 270 may be configured to convert alternating electrical current (AC) to direct electrical current (DC) and to deliver electrical power to the microphone 272. In the embodiment of FIGS. 2A and 2B, electrical power is provided from a source outside of sensor 250 through wires 262 (through AC current). In alternative embodiments, a battery can be provided inside housing 258.

At least one feedthrough 266 is preferably provided for passing electrical wires 262 to through housing 258. Feedthrough 266 is preferably provided through closure 260. In certain embodiments, feedthrough 266 is formed in closure 260; in other words, they are unitary.

Electrical wires 262 may be configured to pass electrical power to implantable device 250. Wires 262 may be configured to transmit the processed microphone signal to the exterior of sound sensor 250. In the latter case, electronic circuit 270 may be configured to modulate the signal on the power wires.

In an alternative embodiment, the transmission is wireless. In such embodiments, the implantable device 250 may be provided with an electromagnetic antenna (not shown).

Rod 256 is an elongate member suitable for coupling membrane 248 to a vibrating structure of the ear. Alternatively, the sound sensor 250 may comprise one or more brackets 254 for additionally connecting membrane 248 to a structure of the middle or inner ear. In certain embodiments, rod 256 or bracket 254 may be coupled to the tympanic membrane, and bracket 254 may be a bracket similar to those used for tympanoplasty. That is, bracket 254 may comprise a disc for coupling to the tympanic membrane. Additionally, in other embodiments, rod 256 or bracket 254 may be coupled to the malleus, the incus, or the stapes, and bracket 254 may comprise, for example, a bracket such as those used for stapedioplasty. In such embodiments, bracket 254 comprises a clip for coupling to one of those structures. In still other embodiments, rod 256 or bracket 254 may be coupled to the elliptical window, round window, the horizontal canal, the posterior canal or the superior canal.

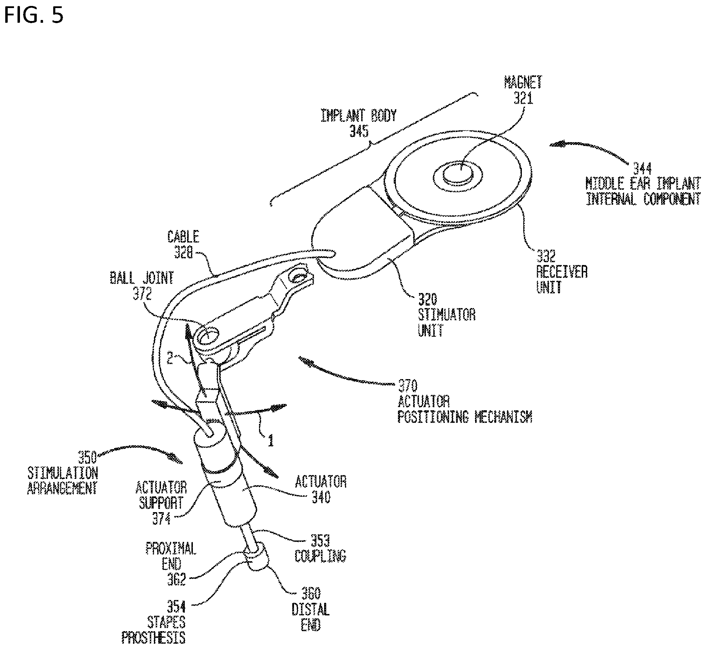

FIG. 5 is a perspective view of an exemplary internal component 344 of a middle ear implant which generally represents internal components 244A and 244B described above. Internal component 344 comprises an internal receiver unit 332, a stimulator unit 320, and a stimulation arrangement 350. As shown, receiver unit 332 comprises an internal coil (not shown), and preferably, a magnet 320 fixed relative to the internal coil. As would be appreciated, stimulator unit 320 is typically hermetically sealed within a biocompatible housing.

Stimulator unit 320 is connected to stimulation arrangement 350 via a cable 328, which includes electrical leads therein (not shown, but inside the sheathing of the cable 328 - in some embodiments, there are two electrical leads that are metallic based and are separately sheathed, and the sheathing is further sheathed by an outer sheathing of the cable - in some embodiments, the electrical leads extend parallel to one another and in other embodiments, the cable is a coaxial cable).

In an exemplary embodiment, cable 328 has a length extending from the implanted body 345 to the actuator 340 of less than, more than, or equal to 0.5, 0.6, 0.7, 0.8, 0.9, 1, 1.25, 1.5, 1.75, 2, 2.25, 2.5, 2.75, 3, 3.25, 3.5, 3.75, 4, 4.25, 4.5, 4.75, 5, 5.25, 5.5, 5.75, 6, 6.25, 6.5, 6.75, 7, 7.5, or 8 or more inches, or any value or range of values therebetween in 0.1 inch increments (e.g., 1.1 inches, 7.8 inches, 3.2 inches to 5.6 inches, etc.). (The length of cable 162 can have these dimensions as well. It is noted that embodiments can use the actuator and the tube microphone as a totally implanted arrangement (e.g., instead of FIG. 3 being a cochlear implant, the cochlear implant is replaced with a middle ear implant).

Stimulation arrangement 350 comprises an actuator 340, a stapes prosthesis 354, and a coupling element 353. A proximal end of stapes prosthesis 354 (if present - other embodiments attached to other parts of the ossicles) is connected to actuator 340 via coupling element 353, and in operation, signals from the stimulator unit 320 are sent through the electrical leads to actuate the actuator 340 so that it vibrates stapes prosthesis 354. Middle ear implant internal component 344 further includes actuator positioning mechanism 370 for positioning actuator 340 and thereby positioning stapes prosthesis 354. The actuator positioning mechanism may be attached to a fixation system (not shown) secured directly to a bone. In one embodiment, the fixation system comprising a cross-shaped component. The fixation mechanism may be secured via one or more bone screws drilled into the recipient's skull through apertures located towards the distal ends of the cross-shaped component. The positioning mechanism may contain one or more position/orientation adjustment components each of which commits relative adjustments in position and/or orientation. In FIG. 5, a position/orientation adjustment component in the form of a ball joint 372 permits the articulation of actuator 340 relative to middle ear implant internal component 344. Actuator support 374 is depicted as being in the form of a collar, and receives and otherwise holds actuator 340 therein, and thus holds the actuator to the actuator positioning mechanism 370. Actuator support 374 allows for longitudinal positioning of the actuator 340 within the actuator support 374.

As may be appreciated by reference to FIG. 5, the position of stapes prosthesis 354 may be adjusted by moving actuator 340 and thereby moving stapes prosthesis 354 because it is rigidly coupled to actuator 340 by coupling 353.

In an exemplary embodiment, a magnetic field that has origins from outside the cable 328, such as by way of example only and not by way of limitation, that which results from magnetic resonance imaging, can result in the generation of a signal within the electrical conduits (leads, coaxial cable, etc.) of cable 328. This as distinguished from the inductance field that can be generated from an external component that is based on sound captured by a microphone of the external component, where the external component is located proximate the receiver unit 332, which inductance field interacts with the coil of the receiver unit 332, the interaction of which generates a signal that is provided to the stimulator unit, where the actuation of the actuator 340 is based thereupon. In some embodiments, the inductance field powers the actuator. That is, the electricity that is utilized to move the actuator to evoke a hearing percept based on the captured sound is generated via the inductance field. In the absence of the inductance field, in at least some exemplary embodiments, the actuator would not have power to actuate, irrespective of whether or not there is an ambient sound. Note also that in some alternate embodiments, such as in a totally implantable hearing prosthesis, where, for example, a microphone is implanted in the recipient, and the implantable prosthesis is essentially entirely self-contained, the implantable component may include a battery, which battery powers the actuator. Still, even in this exemplary embodiment, an external component can be utilized to charge/recharge the battery. This external component can, in at least some exemplary embodiments, generate an inductance field, which inductance field will interact with the receiver unit 332, and generate power which is utilized to recharge the battery. That said, in some alternate embodiments, the battery is a non-rechargeable battery, and has sufficient capacity that the battery can remain implanted for a number of years, such as five or six or seven or eight or nine or 10 or 11 or 12 or 13 or 14 or 15 years or more, at which point at the end of the batteries charge, the battery could be replaced via a relatively minor surgical procedure. It is briefly noted that while the teachings herein are generally detailed with respect to a magnetic field, the teachings herein are also applicable, in some alternate embodiments, to electromagnetic fields that cause the actuation of the actuator / transduction in a transducer. Accordingly, any disclosure herein relating to a magnetic field corresponds to a disclosure of an alternate embodiment associated with an electromagnetic field. Also, it is noted that a device that reacts or otherwise is operated in the face of a magnetic field can correspond to a device that so operates in the face of an electromagnetic field.

It is briefly noted that the above-described effect on the leads can also have a somewhat analogous impact on the lead(s) 162 from the tube mic 150 of FIG. 3, which can result in a signal to the receiver stimulator (and thus might result in activation of the cochlear implant) and/or the generation of a signal to a transducer within the tube mic which moves the working end 152 of the tube mic and thus potentially can result in a hearing percept (if for example the cochlea retains residual hearing), at least for transducers that are of the electromagnetic type and/or piezo type (as opposed to the air gap arrangement of the tube microphone). Accordingly, any disclosure herein associated with a middle ear implant actuator corresponds to a disclosure of an alternate embodiment where the actuator is instead a transducer.

In any event, the point is that the magnetic field at issue that interacts with the electrical conductor and components of the cable 328 is different than the inductance field that is utilized to interact with the receiver unit 332. Further, the magnetic field at issue is a magnetic field that influences the implantable component due to interaction with the cable, as opposed to interaction with the receiver unit. To be clear, in some exemplary scenarios, the magnetic field at issue can also interact with the coil of the receiver unit. However, at issue here, for at least this exemplary embodiment, is the effects of the interaction of that magnetic field with the cable 328.

In this regard, in at least some exemplary embodiments, an external magnetic field can result in a voltage being induced on the electrical conducting components of the cable 328 connecting the actuator with the implanted body 345. In an exemplary embodiment, this induced voltage can be the result of a changing magnetic field and/or radiofrequency fields, with the gradient field as the dominating contributor. In at least some exemplary embodiments, the aforementioned voltages that are induced in the electricity conducting components can result in movement of the actuator. In an exemplary embodiment, such can result in undesired stimulation of the recipient, such as an undesired hearing percept being induced in the recipient. Such an exemplary scenario can occur in an exemplary embodiment where a changing magnetic field operates in audible frequencies in at least some exemplary scenarios.

Accordingly, in at least some exemplary embodiments of the implantable component of figure 5, there is contraindication with respect to exposing a recipient of the implant to an MRI field. In an exemplary embodiment, there is contraindication with respect to exposing the recipient of the implant to an MRI field that is greater than 0.5, 0.75, 1, 1.25, 1.5, 1.75, 2, 2.25, 2.5, 2.75, 3, 3.25, 3.5, 3.75, 4, 4.25, 4.5, 4.75, 5, 5.5, 6, 6.5, 7, 8, 9, 10, 11, 12, 13, 14 or 15 T field or any value or range of values therebetween in 0.1T increments.

The above said, in at least some exemplary embodiments, changes to the design of the implant can be made that make the implant more compatible with exposure to an MRI magnetic field. By way of example only and not by way of limitation, twisted wire leads / a twisted pair is utilized in cable 328. In an exemplary embodiment, this can have utilitarian value with respect to limiting/reducing or otherwise eliminating radiofrequency induced voltages. Still further, in an exemplary embodiment, the design of the implant can be configured so as to limit/reduce and/or eliminate gradient induced voltages. By way of example only and not by way of limitation, a feedback-based arrangement can be utilized. In some embodiments, such an arrangement can at least partially address the gradient induced voltages, while in other embodiments, such can at least partially address the RF frequency induced voltages.

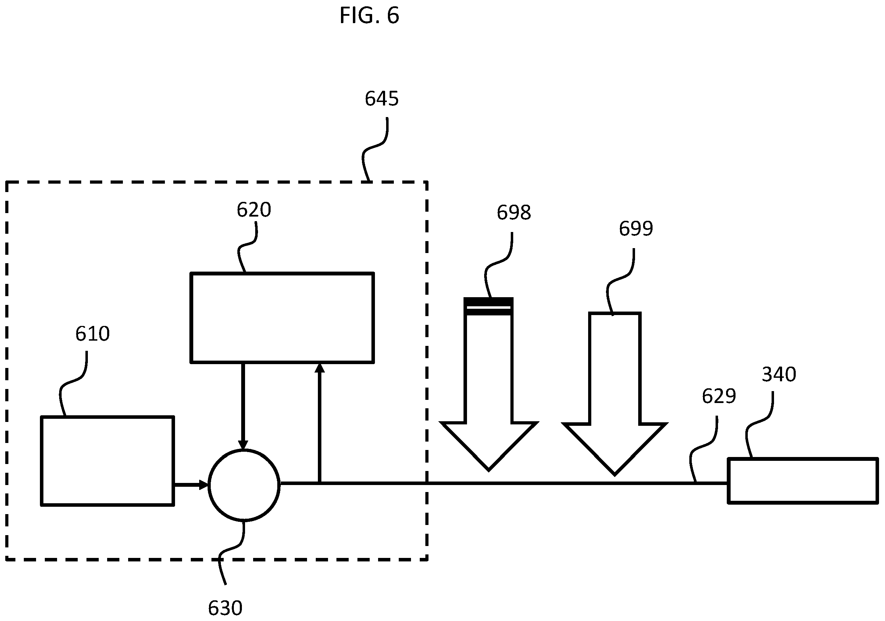

FIG. 6 presents an exemplary functional schematic of an exemplary implantable prosthesis. In this exemplary embodiment, actuator 340 is the middle ear actuator detailed above, and can be an electromagnetic actuator and/or a piezoelectric actuator or any other actuator that is actuated via the application of an electrical signal. Briefly, while the embodiments above and herein focus upon a middle ear implant. In some alternate embodiments, the teachings detailed herein can be applicable to other types of implantable components, such as by way of example only and not by way of limitation, active transcutaneous bone conduction devices. Accordingly, any disclosure herein of a middle ear implant corresponds to a disclosure of an alternate embodiment associated with an active transcutaneous bone conduction device, whether partially implanted or a totally implantable system, however powered. Note also that in at least some exemplary embodiments, the teachings detailed herein are not limited to only hearing prostheses. In an exemplary embodiment, the teachings detailed herein can be applicable to other types of implanted devices, such as other types of medical devices that have an actuator that actuates, which actuator is implanted in the body. Thus, in an exemplary embodiment, the functional schematic of figure 6 can also represent an active transcutaneous bone conduction device, and in other exemplary embodiments, the functional schematic of FIG 6 can also represent another type of prostheses or otherwise another type of implanted medical device. Any implantable arrangement where the teachings detailed herein can have utilitarian value can be the subject of at least some exemplary embodiments, and thus at least some exemplary embodiments include combination of the teachings detailed herein with such.

In any event, whatever actuator corresponds to actuator 340, as can be seen, there is an electrical lead assembly 629 that extends from the implanted body 645, which can be a receiver and/or a receiver stimulator, to actuator 340. As shown, RF frequency field of an MRI magnetic field interacts with the electrical lead assembly 629, as conceptually represented by arrow 699. Also as shown, gradient field of an MRI magnetic field interacts with the electrical lead assembly 629, as conceptually represented by arrow 698. In this exemplary embodiment, at least one of the fields, such as the gradient field 698, induces a voltage in the lead assembly 629. This voltage results in electrical current flowing along lead 629. In at least some exemplary embodiments, without the teachings detailed herein, this can result in the actuation of actuator 340, which can result in stimulation to the recipient, such as, for example, stimulation that can result in a hearing percept. In an exemplary embodiment, gradient field 698 can be a field that has a frequency that is less than, greater than, or equal to 1000, 1500, 2000, 2500, 3000, 3500, 4000, 4500, 5000, 5500, 6000, 6500, 7000, 7500, 8000, 9000, 10,000, 11,000, 12,000, 13,000, 14,000, 15,000, 16,000, or more or any value or range of values therebetween in 1 Hz increments.

In this exemplary embodiment, the induced voltage travels towards the implant body 645. In this exemplary embodiment, the implantable component is provided with a processor 620 that is configured to receive the resulting signal/voltage, process that signal, and output a first signal to mixer 630. This first signal will be combined at adder 630 (or subtractor) with a second signal from digital sound processor 610, which can represent a "clean" signal that is based on captured sound (essentially, the signal that would be outputted by the digital sound processor in the absence of the teachings detailed herein to evoke a hearing percept based on the captured sound). Adder 630 adds and/or subtracts from the second signal, in order to counteract the induced voltages. Thus, the actuator 340 is ultimately presented with a "clean" or at least a "cleaner" signal than that which would be the case in the absence of the feedback (or feedforward) arrangement.

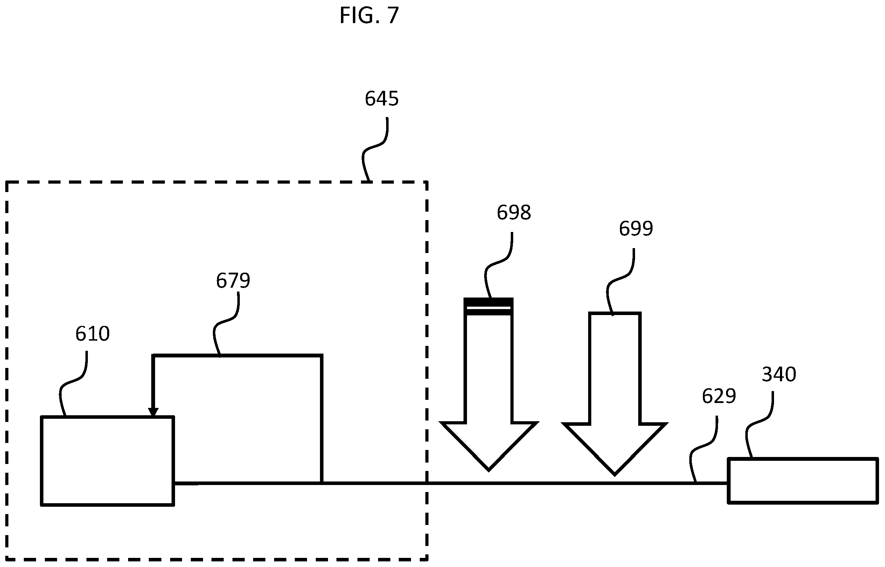

In an exemplary embodiment, processor 620 is configured to measure voltages and/or measure frequency and/or measure another parameter that is utilitarian to executing one or more of the method actions detailed herein and/or to enable one or more of the functionalities detailed herein and output a signal to counteract such. In an exemplary embodiment, the outputted first signal can be an inverted signal and/or a signal that is out of phase in a manner such that the outputted first signal would cancel out, at least partially, the signal induced by the gradient field. In an exemplary embodiment, the processor 620 can include memory and/or can have access to memory that is also located in the implant body 645 which can include lookup tables or the like so that a given measure voltage and/or frequency can be compared against the lookup table, and an output signal based on the measurement can be selected utilizing the lookup table, and then, based on that selection, the first signal can be outputted having features corresponding to that of the lookup table for that given measurement. In an exemplary embodiment, the processor can have access to a program that can computationally evaluate the measurements and determine what characteristics should be present in the first signal to reduce and/or eliminate or otherwise mitigate the effects of the external magnetic field vis-à-vis the lead assembly 629. In at least some exemplary embodiments, component 620 can be a microprocessor and/or a computer chip that is configured to implement the teachings detailed herein. In an exemplary embodiment, component 620 can be integral with the digital sound processor 615 and/or can be a separate component. Indeed, in some embodiments, the component 620 or otherwise the functionality associated therewith can be integrated with or otherwise be a part of the digital sound processor. By way of example only and not by way of limitation, figure 7 provides a path 679 for a feedback or feedforward loop to the digital sound processor 610, although it is noted that in other embodiments, the lead assembly 629 can provide the conduit for the magnetic field induced voltages to the digital sound processor 610. That said, in an exemplary embodiment, a one-way diode or current restrictor device can be located between the digital signal processor and the lead assembly 629, where the path 679 would bypass such. Indeed, in some embodiments, flow restrictors can be located anywhere that can enable the teachings detailed herein. In some embodiments, path 679 is not present.

The above said, in at least some exemplary embodiments, signal comparison can be utilized. In this regard, the digital sound processor 610 would know what it is outputting, if it is outputting anything, and could compare what it outputted/what it "believes" it outputted to the received signal, and thus comparing the two, can determine the contribution of the magnetic field induced voltages.

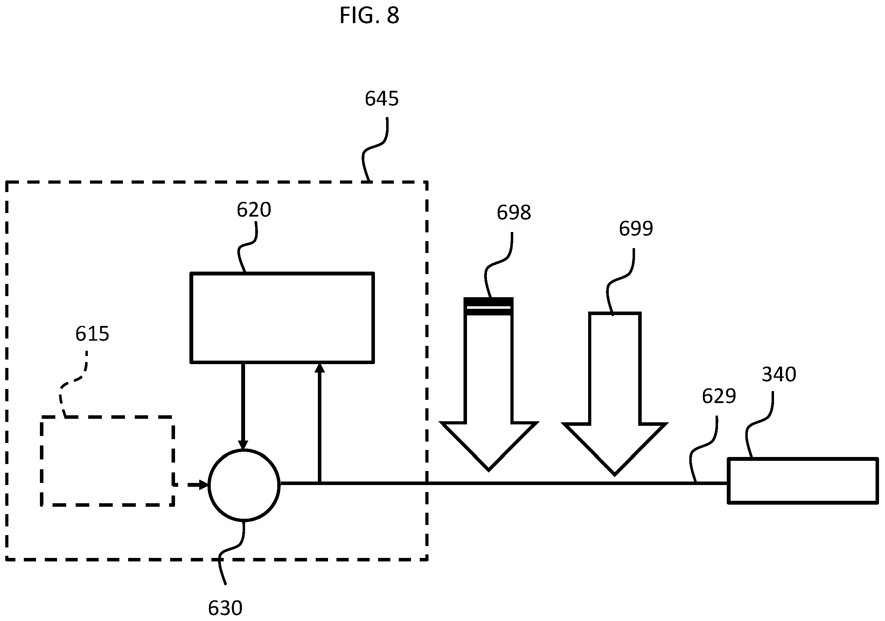

FIG. 8 presents an alternate exemplary embodiment where the digital sound processor 615 may not be present in the implant. Such an exemplary embodiment can be the case with respect to a partially implanted hearing prosthesis where the processing features and the sound capture components are located external to the recipient, and a processed signal based on captured sound is provided via an RF inductance field to the implanted radiofrequency coil, where the signal resulting from the RF inductance field is utilized to actuate the actuator 340. That is, at least some, if not all, of the processing is executed external to the recipient. In this regard, in an exemplary embodiment, instead of processor 615 being present, a radiofrequency coil or antenna, etc., would be present and would be in signal communication with component 630 (intervening componentry can be present, such as signal boosting devices, capacitor devices, etc.). In this exemplary embodiment, component 620 would operate in a manner the same as that detailed above in some embodiments, and first signal could be added and/or subtract into the second signal, where the second signal has its basic origins outside of the recipient.

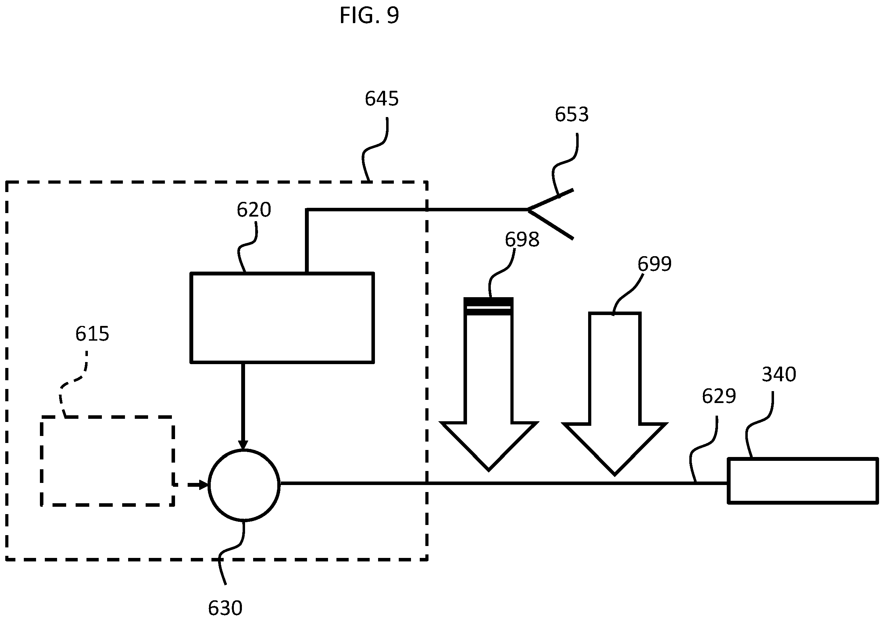

Note further that at least some exemplary embodiments do not utilize a feedback and/or feedforward arrangement and/or can utilize other arrangements to implement the teachings detailed herein. In this regard, figure 9 depicts an exemplary embodiment where an antenna 653 is utilized to obtain measurements associated with the magnetic field that is impinging upon the lead assembly 629. Here, measurements are taken of the field via component 620, and based on the measurements, the first output signal is identified and outputted to component 630. The first output signal interacts with the system in a manner that is analogous to or otherwise the same as the teachings detailed above. By way of example only and not by way of limitation, based on a signal that results from the magnetic field interacting with the antenna 653, the signal can be compared in a lookup table, and based on the lookup table, a first signal can be outputted to component 630.

It is also noted that some embodiments do not necessarily need an antenna 653, at least not one that is outside of the body 645. Any magnetic field sensor that can enable the teachings detailed herein can be utilized in at least some exemplary embodiments. In any event, any device, system, and/or method that can enable a determination of one or more features of the magnetic field (strength, frequency, etc.) that can enable the teachings detailed herein, such as that which can enable the frequency of the magnetic field to be determined and/or that which can enable an estimated first signal to be generated or otherwise outputted to at least partially cancel or otherwise mitigate the signal that results from the interaction of the magnetic field with the lead assembly, can be utilized in at least some exemplary embodiments.

Note also that in some embodiments, there is no magnetic field sensor and/or no feedback/feedforward arrangement. Instead, the prosthesis can be placed into an MRI compatibility mode where a preprogrammed first signal will be generated for a given MRI experience. By way of example only and not by way of limitation, different first signals can be generated for given magnetic field strengths for a given MRI machine, etc. For example, if the magnetic field is a 1.5 Tesla field, a first type of first signal will be outputted by component 620, and if the magnetic field is a 3.0 Tesla field, a second type of first signal will be outputted by component 620 (if the different fields result in different induced voltages). Still further, if the gradient field operates at a given frequency for a given MRI machine, the first signal will be a signal that corresponds to that frequency, and other first signals that are different can be utilized for other gradient field frequencies. In an exemplary embodiment, data can be uploaded to the implant just prior to exposure to the MRI magnetic field for the particular machine and/or for the particular magnetic field, if known.

In some embodiments, the first signal does not necessarily completely cancel out the resulting stimulation, but can cancel out enough of the voltage that is generated as a result of the magnetic field to have utilitarian value.

Again, while embodiments of the teachings detailed herein have focused on component 620 providing a first signal, in some embodiments, the digital sound processor 615 can output the first signal and/or can adjust the output of the second signal to at least partially cancel or otherwise mitigate the signal that results from the magnetic field interacting with the lead assembly 629. That said, the above is applicable to the totally implantable prostheses where the digital sound processor is implanted in the recipient. It is unlikely, at least in some embodiments, that the external component that includes the digital signal processor in a partially implanted prosthesis will be utilized in the MRI machine. Still, if such is done, the external digital signal processor can provide the second signal as modified so as to at least partially cancel the signal induced by the magnetic fields in lead assembly 629.

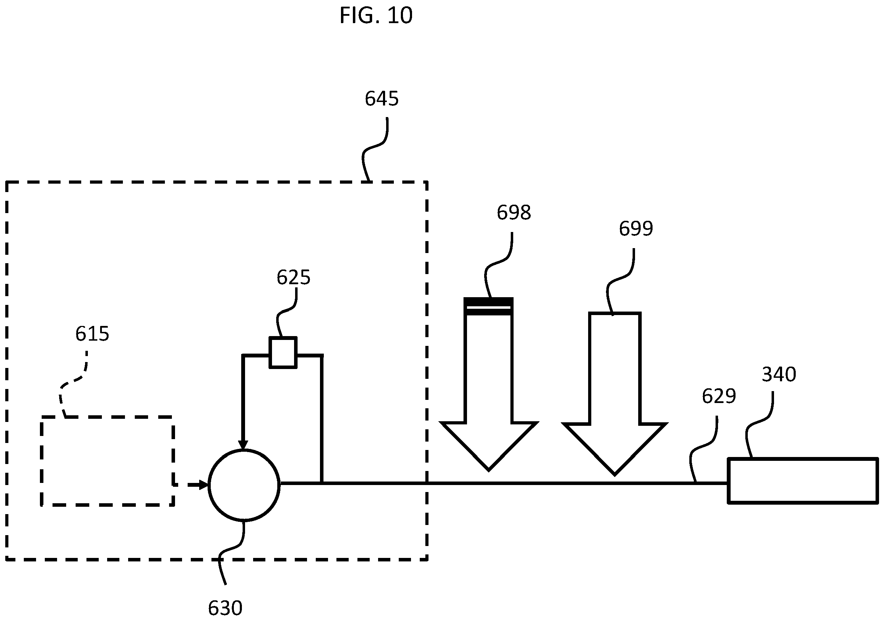

Figure 10 provides an exemplary embodiment where there is no true active processing or the like in the implant, but instead, more simple circuitry is utilized to enable the teachings detailed herein. In this regard, in the exemplary embodiment seen in figure 10, there is a component, such as an inverter or a phase shifting device 625, that receives the signal resulting from the interaction of the magnetic field with the lead assembly 629, and inverts and/or changes the phase of that signal, and provides that modified signal to component 630, where irrespective of any signal received from a digital sound processor, the modified signal at least partially cancels the signal or otherwise mitigates the signal that results from the magnetic field interacting with the lead assembly 629.

Consistent with the embodiment of figure 10, and other embodiments where there is no digital sound processor with the recipient when the recipient is exposed to the MRI field, and/or in scenarios where there is silence (however unlikely), and there is no output from the implanted digital sound processor, there can be scenarios where there is no sound based signal that is being provided to component 630. Accordingly, at least some of the teachings detailed herein include providing the first signal in isolation from any other sound-based signals that are being provided to component 630 and/or to the lead assembly 629. Thus, some embodiments of the teachings detailed herein are executed in combination with evoking a hearing percept based on sound that is captured by the prostheses, while other embodiments of the teachings detailed herein are executed without evoking a hearing percept based on sound that is captured by the prosthesis.

In some embodiments, the teachings associated with the mitigation of the signal that results from interaction of the magnetic field with the electrical lead is executed at a temporal location that is less than or greater than or equal to 0.01, 0.02, 0.03, 0.04, 0.05, 0.06, 0.07, 0.08, 0.09, 0.1, 0.25, 0.5, 0.75, 1, 1.25, 1.5, 1.75, 2, 3, 4, 5, 6, 7, 8, 9, 10, 11, 12, 13, 14, 15,1 6, 171, 8, 19, 20, 21, 22, 23, 24, 25, 26, 27, 28, 29, 30, 45, 60, 100, 120, 150, 200, 300, 400, 500, 600, 1,000, 1,500, 2,000, 2.5k, 3k, 4k, 5k, 6k, 7k, 8k, 9k, 10k, 11k, 12k, 13k, or 14k seconds, or any value or range of values therebetween in 0.01 second increments from the last time that a hearing percept was evoked using the prosthesis based on sound captured by a microphone or other sound capture device of the hearing prosthesis, where such was transduced into a signal to cause a stimulation of tissue of the recipient evoke the hearing percept.

Thus, in view of the above, in at least some exemplary embodiments, the implant is turned on during the MRI scan so that the recipient is able to hear during the MRI scan, while in other embodiments, the implant, while functioning, is not functioning to evoke a hearing percept. With regard to the latter, there can be utilitarian value with respect to putting the implant into an "MRI mode" and not stimulating the recipient during the scan for the purposes of evoking a hearing percept based on ambient sound, but where any stimulation that occurs is a result of the efforts to at least partially cancel or otherwise mitigate the resulting signal from the interaction of the magnetic field with the electrical lead 629 that actuates the actuator 340 at, for example, audible frequencies (which, even if such evokes a hearing percept, is not a hearing percept based on ambient sound). Accordingly, at least some exemplary embodiments include at least partially canceling the induced voltages that result from the magnetic field interaction with the lead assembly 629 without adding a digital sound processor signal to the lead assembly 629, and thus not providing that signal to the actuator 340.

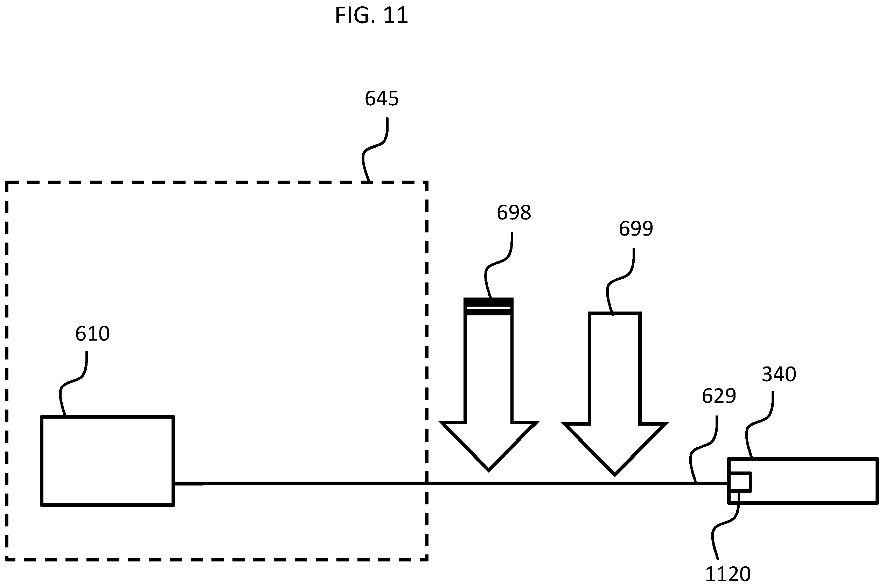

Figure 11 presents another exemplary embodiment of an arrangement that can mitigate the effects of the signal induced in the lead assembly 629. Here, a component 1120 is located downstream of the lead assembly 629 relative to the body 645. In this exemplary embodiment, component 1120 is located inside the actuator 340. That said, in an exemplary embodiment, component 120 can be located outside the actuator. It can be mounted on the actuator were on the lead assembly 629 a bit distant from the actuator 340.

In an exemplary embodiment, component 1120 can be a filter, while in other embodiments, component 1120 breaks the circuit between the lead assembly 629 and the actuated components of the actuator. By way of example only and not by way of limitation, component 1120 can be a switch, while in other embodiments, component 1120 can be a device that increases resistance in a variable manner so as to prevent the signal that is induced via the magnetic field from reaching the actuated components of the actuator.

With respect to the exemplary filter embodiment, in an exemplary embodiment, component 1120, when such corresponds to a filter, can be a variably controlled filter that can variably filter out electrical signals within a certain range of frequencies. By way of example only and not by way of limitation, when the prosthesis is placed into an MRI mode, filter 1120 prevents signals having frequencies between 2kHz and 8kHz from reaching the actuator components of the actuator 340. In some embodiments, the filter can be a narrower filter or a broader filter. In some embodiments, the filter can prevent signals above and/or below or at 750, 1,000, 1,250, 1,500, 1,750, 2,000, 2,250, 2,500, 3,000, 3,500, 4,000, 5,000, 6,000, 7,000, 8,000, 9,000, 10,000, 11k, 12k, 13k, 14k, 15k, 16k, 17k, 18k, 19k, 20k, 21k, 22k Hz or greater, or any value or range of values therebetween in 1 kHz increments.

Accordingly, if the gradient field creates a signal having a frequency of 5kHz, a filter that will not permit signals with frequencies of 3kHz to 7kHz to pass will prevent the signal from actuating the actuator, while the prosthesis permits signals at different frequencies outside that range to reach the actuator, thus permitting the actuator to evoke a hearing percept while the recipient is being exposed to the MRI magnetic field.

That said, in some embodiments, the hearing prosthesis is not configured to evoke a hearing percept when the recipient is in the MRI, such as where, for example, the external component has been removed from the recipient. Thus, in some embodiments, there is a hearing prosthesis that is a partially implantable hearing prosthesis where the implantable portion is configured such that a cancellation signal is present when exposed to the external magnetic field that at least partially cancels the signal resulting from the external magnetic field.

Note also that in some embodiments where the range is relatively broad, and it is desired for the recipient to at least hear ambient sound having the frequencies that are filtered, in some embodiments, in the MRI mode, the digital sound processor can purposely reduce and/or increase the frequencies of ambient sound falling within the range so that the recipient will have a hearing percept of those sounds, albeit at a different frequency. That is, for example, if a speaker has a voice frequency that has much content around 4 kHz, the digital sound processor will reduce that to 2 kHz, and output a signal accordingly, which will get through the filter albeit the evoke a hearing percept will sound much lower than that which would otherwise be the case.

When the recipient is done with the MRI, component 1120 will be deactivated, and the prosthesis will work accordingly.

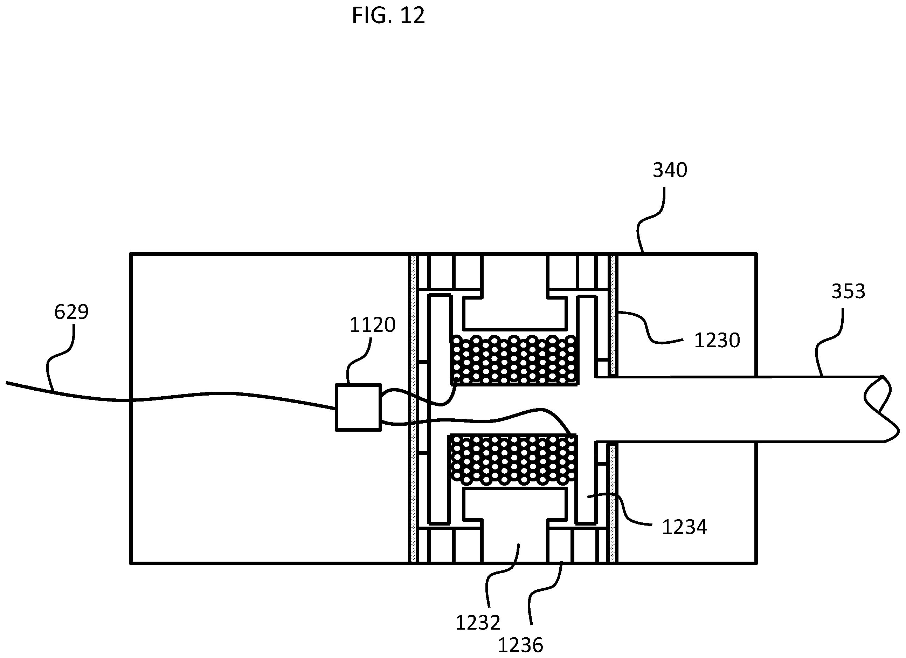

FIG. 12 depicts a cross-sectional view of the actuator 340, showing that the actuated component of the actuator is an electromagnetic actuator with a coil wrapped around a bobbin 1234, and permanent magnets 1236 straddling the yoke 1232, where the bobbin 1234 is supported by Springs 1230 which are mounted against the interior housing wall of the housing of the actuator 340, and where the coupling element 353 moves with movement of the bobbin 1234 when the coil is energized. When an alternating current is applied via the electrical lead assembly 629, the bobbin will move back and forth relative to the housing, and thus move the coupling element 353 back and forth, which in turn will move the portion of the ossicles and/or the portion of the round window or oval window or other anatomical component to evoke a hearing percept.

As can be seen in figure 12, component 1120 is interposed between the lead assembly 69 and the coil that is wrapped around the bobbin 1234. Component 1120 is located in the housing of the actuator 340, and can operate as detailed above. Thus, with the embodiment of FIG. 12, and induced current in the lead assembly 629 can be prevented from reaching the coil of the actuator, at least in an alternating manner, and thus the actuation will be prevented.

Note that consistent with the above, the teachings herein are not limited to electromagnetic actuators as seen in FIG. 12. Instead, the leads could lead to a piezoelectric actuator or any other component that can cause actuation. Moreover, the leads could lead to another type of stimulation device.

In view of at least some of the embodiments above, it can be seen that in an exemplary embodiment, there is an apparatus comprising an implantable portion of a hearing prosthesis, wherein the apparatus is configured to at least partially cancel a signal in the implantable portion resulting from an external magnetic field generated external to a recipient of the hearing prosthesis. In an exemplary embodiment, the signal is a signal generated by the external magnetic field interacting with an electrical lead extending between a stimulation output device (e.g., actuator 340) of the prosthesis and a stimulator (e.g., DSP 610) and/or receiver (e.g., inductance coil) of the implantable portion.

In some embodiments, the implantable portion of the hearing prosthesis includes an actuator and electrical leads extending from the actuator, and the actuator will actuate due to at least some varying magnetic fields in an overall field that has a value that is greater than at least 1.5T as a result of interaction of the magnetic field with the leads. In an exemplary embodiment, the at least some varying magnetic fields is in a field that is greater than 0.5, 0.75, 1, 1.25, 1.5, 1.75, 2, 2.25, 2.5, 2.75, 3, 3.25, 3.5, 3.75, 4, 4.25, 4.5, 4.75, or 5T, or any value or range of values therebetween in 0.1T increments and/or has a frequency that is less than, greater than and/or equal to 500, 1,000, 1,500, 2,000, 2,500, 3,000, 3,500, 4,000, 4,500, 5,000, 5,500, 6,000, 6,500, 7,000, 7,500, 8,000, 9,000, 10,000, 11,000, 12,000, 13,000, 14,000, 15,000, 16,000, 17,000, 18,000, 19,000, 20,000, 21,000, 22,000 or more, or any value or range of values therebetween in 1 Hz increments. The varying magnetic field can have a maximum gradient value of between 10 to 75 mT, or, for example, can have a value of 1, 2, 3, 4, 5, 6, 7, 8, 9, 10, 11, 12, 13, 14, 15, 20, 25, 30, 35, 40, 45, 50, 55, 60, 65, 70, 75, 80, 85, 90, 95, or 100 mT or more, or any value or range of values therebetween in 0.1 mT increments.

In some embodiments, the signal resulting from the external magnetic field is an alternating current that has component(s) having frequencies less than 100 kHz, 80 kHz, 60 kHz, 50, 40, 30, 29, 28, 27, 26, 25, 24, 23, 22, 21, 20, 19, 18, 17, 16, 15, 14, 13, 12, 11, 10, 9, 8, 7, 6, 5, 4, 3, 2, or 1 kHz or lower, or any value or range of values therebetween in 1 Hz increments and/or less than 500, 450, 400, 350, 300, 250, 200, 150, 100, 90, 80, 70, 60, 50, 40, 30, 25, 20, 19, 18, 17, 16, 15, 14, 13, 12, 11, 10, 9, 8, 7, 6, 5, 4, 3, 2, or 1 mV, or any value or range of values therebetween in 0.1 mV increments. In an exemplary embodiment, the components having the aforementioned features constitute at least about 10, 15, 20, 25, 30, 35, 40, 45, 50, 55, 60, 65, 70, 75, 80, 81, 82, 83, 84, 85, 86, 87, 88, 89, 90, 91, 92, 93, 94, 95, 96, 97, 98, 99, or 100 percent, or any value or range of values therebetween in 1% increments of the total energy in the lead generated by the magnetic field.

It is noted that in at least some exemplary embodiments, the signal can have frequencies that are larger than the aforementioned frequencies and/or magnitudes. It is just that the signals must have the aforementioned values in some embodiments. To be clear, in at least some exemplary embodiments, there are frequencies and voltages that are induced as a result of other features of the magnetic field, such as the radiofrequency nature of the magnetic field. In some exemplary embodiments, the teachings detailed herein not address those frequencies/magnitudes that results there from, at least not with respect to the cancellation/mitigation teachings detailed herein associated with the audible frequencies or otherwise that would result in the actuation of the actuator in a manner that would evoke a hearing percept. Still, in other embodiments, the teachings detailed herein, such as the utilization of a twisted pair of leads, can be utilized to address potentially generated signals that are outside the aforementioned ranges, although it is noted that in at least some exemplary embodiments that utilize the twisted pairs, the signal is not generated because the twisted pairs shield against such.

Indeed, in an exemplary embodiment, the teachings detailed herein are not directed towards shielding against the magnetic field and/or preventing the generation of the voltages as a result of interaction of the magnetic field with the implant. In this regard, in at least some exemplary embodiments, the voltages are generated / the signals are generated having the aforementioned features, and the prostheses cancels or otherwise mitigate such.

In some embodiments of the above detailed apparatus, the implantable portion of the hearing prosthesis includes circuitry configured to at least partially cancel the signal generated by the magnetic field. The implantable portion of the hearing prosthesis is further configured with a feedforward and/or feedback circuit that enables the signal to be provided to the circuitry, and the circuitry is configured to at least partially cancel the signal using the output of the feed forward and/or feedback circuit.

In an exemplary embodiment, at least about 25, 30, 35, 40, 45, 50, 55, 60, 65, 70, 75, 80, 81, 82, 83, 84, 85, 86, 87, 88, 89, 90, 91, 92, 93, 94, 95, 96, 97, 98, 99, or 100 percent of the signal is cancelled, where the percentages can be the total magnitude of the signal or the total voltage of the signal, etc. Also, in some embodiments, the aforementioned percentages are for frequencies below 100 kHz, 80 kHz, 60 kHz, 50, 40, 30, 29, 28, 27, 26, 25, 24, 23, 22, 21, 20, 19, 18, 17, 16, 15, 14, 13, 12, 11, 10, 9, 8, 7, 6, 5, 4, or 3 kHz, or any value or range of values therebetween in 1 kHz increments. It is noted that the aforementioned cancellation figures can be for any of the embodiments detailed herein, not just the embodiments that utilize feedback and/or feedforward arrangements. Indeed, consistent with the teachings detailed above, in some embodiments, the feedback and/or feedforward circuits are not necessarily used, and other embodiments, such as the preprogrammed cancellation signal, is utilized. Accordingly, in at least some exemplary embodiments, the implantable portion of the hearing prosthesis is configured to provide a cancellation signal that at least partially cancels the signal resulting from the external magnetic field.

As noted above, at least some exemplary embodiments enable the prosthesis to be utilized to evoke a hearing percept based on ambient captured sound while the signal mitigation is implemented. Thus, in some embodiments, the implantable portion of the hearing prosthesis is configured to evoke a hearing percept based on ambient sound while at least partially cancelling the signal resulting from an external magnetic field. (It is noted that any disclosure herein of partially canceling the signal corresponds to a disclosure in an alternate embodiment of totally canceling the signal, unless otherwise noted. Note also that at least partially canceling the signal includes totally canceling the signal.)

An alternate embodiment does not necessarily involve cancellation of the signal that is generated as a result of the interaction of the magnetic field with the lead 629 and/or preventing the signal from reaching the actuator. Instead, in an exemplary embodiment, there is the action of driving or otherwise providing a signal to the actuator having a frequency component and/or magnitude that has utilitarian value respect to at least partially mitigating the effects of the signal with respect to the generation of a hearing percept.

In an exemplary embodiment, the aforementioned signal induced by the magnetic field reach is the actuator, and this signal would otherwise result in the actuator actuating to evoke a hearing percept, but by operating the actuator according to the teachings detailed herein, at least about 25, 30, 35, 40, 45, 50, 55, 60, 65, 70, 75, 80, 81, 82, 83, 84, 85, 86, 87, 88, 89, 90, 91, 92, 93, 94, 95, 96, 97, 98, 99, or 100 percent of the hearing percept that would otherwise result due to the signal is eliminated, as measured based on the total magnitude of the percept across all frequencies.

In an exemplary embodiment, the signal would otherwise result in the actuator actuating to evoke a hearing percept, and the hearing percept would have an average, across all frequencies, less than, greater than or equal to 50, 55, 60, 65, 70, 75, 80, 85, 100, 105, 110, 115, 120 dB, or any value or range of values therebetween in 1 dB increments.

Thus, in an exemplary embodiment, there is an implantable hearing prosthesis, wherein the implantable hearing prosthesis is configured to function to reduce and/or eliminate a hearing percept that would be evoked by the hearing prosthesis when subjected to an external magnetic field generated external to a recipient of the hearing prosthesis in the absence of the functioning. It is noted by functioning, this is different than disabling the hearing prosthesis and/or breaking a circuit to prevent stimulation / prevent the actuator from operating. In this regard, such would exclude the embodiment of figure 11.

Consistent with the teachings above, the prosthesis can be such that it at least partially cancels a signal generated by interaction of the external magnetic field with the prosthesis. That said, in some alternate embodiments, the prosthesis does not cancel, in part or in whole, the signal. In some embodiments, the prosthesis is configured to generate a signal having a frequency and amplitude that reduces and/or eliminates the hearing prosthesis, where the signal causes actuation of the actuator. In an exemplary embodiment, the prosthesis is configured to generate a low frequency, high amplitude signal so as to reduce and/or eliminate the hearing percept, which signal actuates the actuator of the prosthesis, whether or not such actuation results in a hearing percept (some embodiments may result in such, and some embodiments may not result in such). Still further, in an exemplary embodiment, the prosthesis is configured to generate a high frequency, high amplitude signal so as to reduce and/or eliminate the hearing percept, which signal actuates the actuator of the prosthesis, again whether or not such actuation results in a hearing percept. In some embodiments, the prosthesis is configured to generate a medium frequency, high amplitude signal, so as to reduce and/or eliminate the hearing percept, again which signal actuates the actuator of the prosthesis. (It is noted that such does not require the actuator operate at that frequency, only that the signal be generated, as this is in reference to the signal.)

It is noted by way of example that the generation/application of the aforementioned signals can be executed in a scenario where the ambient sound does not include or otherwise does not effectively include any meaningful components at the frequencies and/or amplitudes associated with the signal that is applied to the actuator. Indeed, in at least some exemplary embodiments, during normal operation of the hearing prosthesis, the hearing prosthesis would not operate at those frequencies even if sound as corresponding to sounds having the frequencies and/or amplitudes existed in the environment. In an exemplary embodiment, the prosthesis is configured to generate the aforementioned signals in the complete absence of any ambient sound having those frequencies and/or amplitudes. Indeed, in an exemplary embodiment, the prosthesis is configured to generate those signals in the complete absence of any input associated with or otherwise based on the ambient sound. That is, in the complete absence of any sound capture, whether because there is no sound or because the microphone is shut down or otherwise removed from the system (such as in the case of a partially implantable hearing prosthesis), the aforementioned signals will still be generated.

In some exemplary embodiments, the prosthesis is configured to generate a signal having a frequency of less than, greater than and/or equal to 0.01, 0.05, 0.1, 0.2, 0.3, 0.4, 0.5, 0.6, 0.7, 0.8, 0.9, 1, 1.1, 1.2, 1.3, 1.4, 1.5, 1.6, 1.7. 1.8, 1.9, 2, 2.25, 2.5, 2.75, 3, 3.25, 3.5, 3.75, 4, 4.25, 4.5, 4.75, 5, 5.5, 6, 6.5, 7, 7.5, 8, 8.5, 9, 9.5, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 45, 50, 55, 60, 65, 70, 75, 80, 90, 100, 110, 120, 130, 140, 150, 160, 170, 180, 190, or 200 Hz, or any value or range of values therebetween in 0.01 Hz, increments, so as to reduce and/or eliminate the hearing percept, which causes the actuator to actuate

In some exemplary embodiments, the prosthesis is configured to generate a signal having a frequency of less than, greater than and/or equal to 15 kHz, 16, kHz, 17, kHz, 18, 19, 20, 21, 22, 23, 24, 25, 26, 27, 28, 29, 30, 31, 32, 33, 34, or 35 kHz or greater, or any value or range of values therebetween in 1 Hz, increments, so as to reduce and/or eliminate the hearing percept, which causes the actuator to actuate.

In some exemplary embodiments, the prosthesis is configured to generate a signal having a frequency of less than, greater than and/or equal to 4, 5, 6, 7, 8, 9, 10, 11, 12, 13, 14, 15, 16, or 17 kHz or greater, or any value or range of values therebetween in 1 Hz, increments, so as to reduce and/or eliminate the hearing percept, which causes the actuator to actuate.

In some exemplary embodiments, the prosthesis is configured to generate a signal having a mean or median or maximum amplitude that is at least 30, 35, 40, 45, 50, 55, 60, 65, 70, 75, 80, 85, 90, 95, or 100%, or any value or range of valuates therebetween in 1% increments of the maximum amplitude that can be generated for actuation of the actuator based on ambient sound (e.g., very loud sounds), so as to reduce and/or eliminate the hearing percept. In an exemplary embodiment, the maximum amplitude of the signal that can be generated to actuate the actuator is less than, greater than or equal to 0.3, 0.4, 0.5, 0.6, 0.7, 0.8, 0.9, 1, 1.1, 1.2, 1.3, 1.4, 1.5, 1.6, 1.7, 1.8, 1.9, 2, 2.25, 2.5, 2.75, 3, 3.5, 4, 4.5, 5, 5.5, 6, 6.5, 7, or 8 V, or any value or range of values therebetween in 0.01 V.

In at least some exemplary embodiments, the amplitude of the signal that is generated and provided to the actuator is at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, 60, 70, 80, 90, 100, 125, 150, 175, 200, 250, 300, 350, 400, 500, 600, 700, 800, 900, or 1,000 or more times the amplitude of the signal that is generated as a result of exposure of one or more of the aforementioned magnetic fields to the electrical leads.

According to the invention the prosthesis is configured to dampen stimulation that results in the hearing percept via functioning of the prosthesis in an MRI mode. In an exemplary embodiment, the prosthesis is configured to mask an MRI induced voltage to reduce and/or eliminate the hearing percept that results from the MRI induced voltage.

In an exemplary embodiment, by moving the actuator at the aforementioned frequencies and at the magnitudes corresponding to the aforementioned amplitudes, in at least some exemplary embodiments, this has the result of damping the stimulation. By operating the actuator at certain frequencies coupled with certain magnitudes, the hearing percept that would otherwise exist owing to the signal that results from the magnetic field interacting with the electrical leads is dampened.

In an exemplary embodiment, such as where, for example, the actuator is moved at a low frequency, such as for example, 1 Hz, without being bound by theory, the resulting signal that is generated by the magnetic field interacting with the leads is essentially drowned out and no hearing percept at the frequency of the magnetic field results or otherwise the hearing percept that results at those frequencies is reduced relative to that which would otherwise be the case. In an exemplary embodiment, there is little to no hearing percept that is evoked at the frequency at which the actuator is driven (e.g., the 1 Hz). Still further, in an exemplary embodiment, such as where, for example, the actuator is moved at a high frequency, such as 25,000 Hz, without being bound by theory, the resulting signal that is generated by the magnetic field interacting with the leads is essentially drowned out and no hearing percept at the frequency of the magnetic field results or otherwise the hearing percept that results at those frequencies is reduced relative to that which would otherwise be the case. In an exemplary embodiment, there is little to no hearing percept that is evoked at that frequency at which the actuator is driven.

It is noted that any of the reductions detailed herein can correspond to any embodiment unless otherwise noted.

Still further, in an exemplary embodiment, also without being bound by theory, the signal that is applied to the leads at the aforementioned frequency and amplitude is such that the signal that results from the magnetic field interacting with the electrical leads has little to no impact on the actuation of the actuator because the signal having the higher amplitude overwhelms the other signal, and thus little to no hearing percept is evoked at the frequencies of the magnetic field and/or at any hearing percept that exist is reduced relative to that which would otherwise be the case. Moreover, again consistent with the teachings of the above, in an exemplary embodiment, there is little to no hearing percept that is evoked at the frequency at which the signal is applied.

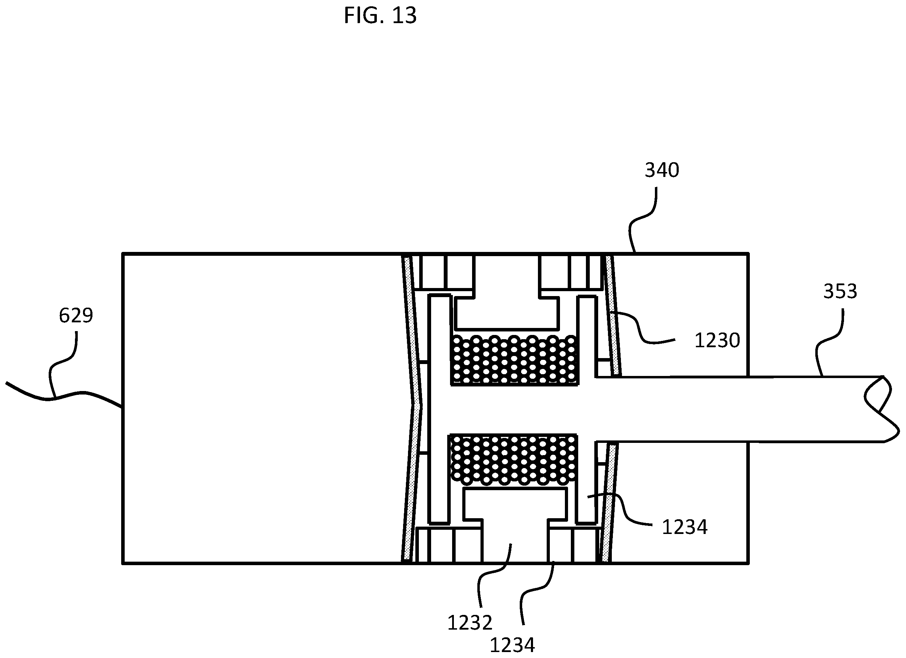

Note further that in at least some exemplary embodiments, instead of an alternating current that is applied to the actuator, a direct current is applied (it is noted that any disclosure of a signal with a frequency corresponds to an alternating current unless otherwise noted). In this regard, in an exemplary embodiment, the prosthesis is configured to provide an alternating current to the actuator to actuate the actuator to evoke a hearing percept during normal operation. In at least some exemplary embodiments, the alternating currents is applied at the frequency of the sound that is captured, or at least otherwise related to the sound that is captured. Conversely, when implementing the functionality of the prosthesis to reduce and/or eliminate the hearing percept that results from the frequency that interacts with the electrical leads, the prosthesis is configured to apply a direct current to the actuator. Thus, in an exemplary embodiment, such as where the prosthesis includes an actuator, the prosthesis can be configured to apply a current to the actuator to urge a moving part of the actuator to and/or towards a given location to reduce and/or eliminate the hearing percept. In this regard, FIG. 13 depicts an exemplary scenario where the prosthesis functions to do exactly that. As can be seen, the prosthesis has driven the moving component of the actuator forward, and that location is held or otherwise generally maintained by maintaining a direct current through the coil. It is noted that the current can be reversed and maintained, and thus, in at least some embodiments, the location depicted in figure 13 would be reversed (the springs would be deflected away from the working end instead of being deflected towards the working end). In any event, whatever the results, in at least some exemplary embodiments, providing that the moving component of the actuator is held in place or otherwise is prevented or otherwise limited from moving while the magnetic field is generating the signal, a hearing percept is reduced and/or prevented from occurring because the actuator cannot actuate, at least not completely.

In an exemplary embodiment, the application of the electrical current effectively locks out the actuator. Note that in the embodiment of figure 13, there is no mechanical locking mechanism. Instead, the prosthesis is functioning to lockout the actuator. This as opposed to an embodiment where a mechanical lock is used. Indeed, in an exemplary embodiment, a second actuator could be located within the actuator that would actuate to move a lock and thus prevent the actuator from actuating. However, that would not correspond to causing the hearing prosthesis to function to reduce and/or eliminate the hearing percept. That is, such a locking device would not correspond to an implantable hearing prosthesis that is configured to function to reduce and/or eliminate a hearing percept that would be evoked by the hearing prostheses when subjected to an external magnetic field generated external to a recipient of the hearing prostheses in the absence of the functioning. That said, in an alternate embodiment, instead of this feature or in addition to this feature, a mechanical locking device can be also included in at least some exemplary embodiments.

In an exemplary embodiment, the prosthesis is configured to function to saturate the electromagnetic actuator with respect to the dynamic magnetic flux such that the actuator will not respond or otherwise responds relatively weakly to the signal generated by the magnetic field. By way of example only and not by way of limitation, a very high amplitude current can be applied that creates a very strong / high magnetic flux that results in any dynamic magnetic flux that results from the signal that is generated as a result of the magnetic field interacting with the lead being *de minimis* or otherwise reducing and/or eliminating any hearing percept that would be evoked because of such.

In at least some exemplary embodiments, there is an apparatus, comprising an implantable prosthesis, wherein the implantable prosthesis is configured to provide a signal to an actuator of the prosthesis to actuate the actuator at a different frequency and/or amplitude relative to a phenomenon induced signal induced in the prosthesis that otherwise results in a respective actuation frequency and/or amplitude induced by a phenomenon unrelated to the prosthesis while the phenomenon is present, wherein the signal to actuate the actuator at the different frequency and/or amplitude at least partially mitigates the effects of the phenomenon induced signal.

In at least some embodiments, the phenomenon is a magnetic field of an MRI machine that generates at least a 1T magnetic field (or any of the fields detailed herein), and the implantable prosthesis is a middle ear implant (again, not all embodiments are directed to middle ear implants, or hearing prostheses for that matter), and the prosthesis is configured to operate to accommodate the magnetic field gradient of the MRI field with respect to the influence thereof on the prosthesis, thereby at least partially mitigating the effects of the phenomenon induced signal. In some embodiments, the provided signal is a signal that results in a frequency of actuation of the actuator that is at least one of above 15 kHz or below 30 Hz (or any of the values detailed herein as discussed above). In at least some embodiments, the provided signal is a signal that is at least 0.2, 0.3, 0.4, 0.5, 0.6, 0.7, 0.8, 0.9, 1, 1.25, 2.5, 2.75, 2, 2.5, 3, 3.5, 4, 4.5, 5, 5.5, 6, 6.5, 7, 8, 9, or 10 V, or any value or range of values therebetween in 0.01V increments and the actuator is configured to operate to evoke a hearing percept having a maximum amplitude at at least 2, 3, 4, 5, 6, 7, 8, 9, 10, 11, 12, 13, 14, or 15 times the voltage provided.

Further, as can be seen from the above, the implantable prosthesis can be configured to provide an alternating signal to the actuator and enable voltages induced via the phenomenon to be superimposed onto the alternating signal, thereby at least partially mitigating the effects of the phenomenon induced signal. Further, the implantable prosthesis can be configured to dampen the phenomenon induced signal in the absence of external stimulus.

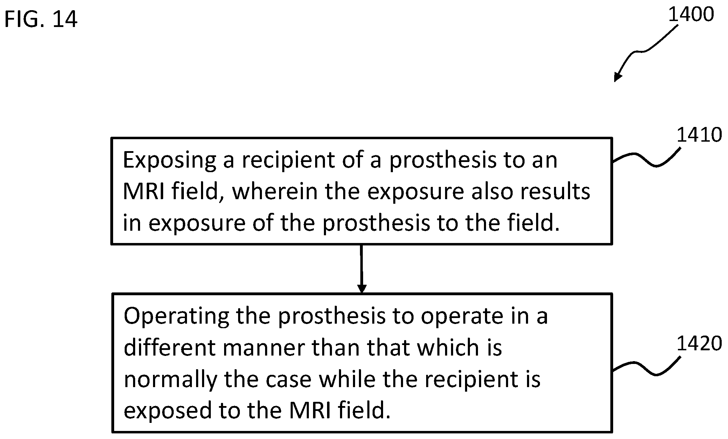

Embodiments include methods. In this regard, FIG. 14 provides an exemplary flowchart for an exemplary method, method 1400, which includes method action 1410, which includes exposing the recipient of a prosthesis to an MRI field. In this exemplary embodiment, the exposure also results in exposure of the prosthesis to the field. The field can be any of the fields detailed herein or variations thereof. Method 1400 also includes method action 1420 which includes operating the prosthesis to operate in a different manner than that which is normally the case while the recipient is exposed to the MRI field. It is noted that the action of operating is different than the embodiment of breaking the circuit as detailed above with respect to figure 11. However, variably filtering the signal according to that embodiment would constitute operating.

Consistent with the teachings above, the action of operating the prosthesis can result in an output by the prosthesis of a low frequency, high amplitude output. Here, the actuator operates. Also, consistent with the teachings detailed above, the action of operating the prosthesis results in an output by the prosthesis of a high frequency, high amplitude signal. In an exemplary embodiment, such as where the prosthesis is a hearing prosthesis, the action of operating the prosthesis in a different manner than that which is normally the case can correspond to doing something with the hearing prostheses that also corresponds to operation of the hearing prostheses that would not otherwise be done when the hearing prosthesis is being utilized to evoke a hearing percept based on captured ambient sound. For example, the utilization of the feedback and/or feedforward circuit to cancel at least a portion of the signal in the electrical leads would not normally be done. Still further by example, the operation of the actuator at the above-noted frequencies and amplitudes would also not be done, at least in a scenario where the ambient sound does not include or otherwise does not effectively include any meaningful components at the frequencies and/or amplitudes associated with the signal that is applied to the actuator. Again, as noted above, in an exemplary embodiment, the prosthesis is configured to generate the aforementioned signals in the complete absence of any ambient sound having those frequencies and/or amplitudes. In an exemplary embodiment, method action 1420 can include operating the prosthesis such that output by the prosthesis of a low frequency high amplitude output exists or otherwise results even though there is no ambient sound that would result in the actuator actuating at those frequencies and/or amplitudes. Again, in some embodiments, the actuator can be actuated in the complete absence of any input related to ambient sound. Thus, in an exemplary embodiment, method action 1420 can be executed with a hearing prosthesis where the hearing prosthesis is not receiving any input indicative or otherwise based on ambient sound.

In an exemplary embodiment, the action of operating the prosthesis according to method action 1420, such as where the prosthesis is a hearing prosthesis, is executed such that the hearing prosthesis actuates the actuator even though there is no ambient sound that exists that would otherwise cause the actuator to actuate in such a manner and/or even though the ambient sound that is present would cause the prosthesis to actuate the actuator in a different manner than that which is the case if the prostheses was operated in a normal manner.

Consistent with the teachings detailed herein, method action 1420 can at least partially counteract the effects of the MRI field on the prosthesis.

In at least some exemplary embodiments, the action operating the prosthesis results in at least one of the elimination of or a reduction in a loudness of a hearing percept evoked by the prosthesis due to interaction of the MRI field with the prosthesis by at least 10, 15, 20, 25, 30, 35, 40, 45, 50, 55, 60, 65, 70, 75, 80, 85, 90, 95, or 100% relative to that would otherwise be present in the absence of the action of operating the prosthesis. In an exemplary embodiment, loudness is the total amplitude of all frequency components, while in other embodiments, it is a mean or median or mode of the amplitude of all frequency components.

An exemplary embodiment includes a saturation technique that involves the prosthesis saturating the middle ear of the recipient. Thus, in an exemplary embodiment, the prosthesis is configured to saturate a middle ear of the recipient when functioning to reduce and/or eliminate the hearing percept. By way of example, there can be an MRI mode into which the prosthesis can be entered where high amplitude, low frequency infrasound is used to stimulate the patient during MRI. This low frequency sound can be inaudible by the patient, and the high amplitude may cause a saturation of the middle ear. The voltages induced during MRI can be superimposed to this dominating low frequency signal, resulting in a dampened audible stimulation for the patient. In an alternate embodiment, there can be an MRI mode into which the prosthesis where high frequency infrasound is used (high amplitude can be used therewith, but in other embodiments, the amplitude can be lower).

Consistent with some of the teachings herein, in an exemplary embodiment, the prosthesis includes an electromagnetic actuator and the action of operating the prosthesis magnetically saturates at least a portion of the actuator. This can prevent or at least limit actuator movement when the lead(s) experience the magnetic field and a voltage that would otherwise result in movement of the actuator that would cause a hearing percept because of the saturation of the magnetic component(s) of the actuator. Saturation can be achieved by any means.

In an exemplary embodiment, the MRI mode can include the application of a very high frequency alternating current that prevents or limits actuator movement, thus at least reducing, if not eliminating, the hearing percept that results from the voltage that is induced in the lead(s). Alternatively, a very strong direct current can be applied.

In an exemplary embodiment, there is an implantable prosthesis that is configured to actuate to purposely trigger a stapedius reflex in a repeatable manner to at least partially mitigate the effects of the phenomenon induced signal. In this regard, an MRI mode can be present where the prosthesis is actuated to trigger a stapedius / acoustic reflex. This can occur repeatedly or can be maintenance of such, or at least maintain such for longer than that which is normal. The actuator (or more accurately, the prosthesis) can mimic input that would result in the reflex.

In an exemplary embodiment, depending on the arrangement of the connection of the actuator with tissue of the recipient, the actuator can be configured or otherwise controlled by the prosthesis to pull the stapes or a component connected thereto away from the oval window of the cochlea or otherwise have the tensor tympani muscle stiffen the ossicular chain and/or pull the malleus in toward the middle ear or otherwise duplicate such action. This can be done in a manner that maintains the reflex / reflex features for a sufficient duration and/or in a repeated manner, providing that such at least partially results in blockage / limitation of the hearing percept that would otherwise result as a result of the voltage induced in the leads from the MRI magnetic field. Programming in the prosthesis can enable the control of the actuator to mimic the input that results in the reflect (or partially results in the reflex).

To be clear, while this embodiment at least partially mimics the stapedius reflex / results thereof, the goal is not necessarily to protect the organ of Corti (although if such happens, that is good as well). The goal is to reduce / eliminate the hearing percept that results from the induced voltages in the leads. Thus, the resulting "movements" or "positioning" of the components of the middle ear may not necessarily be that which would correspond to that which results from the stapedius reflex, providing that those movements ultimately reduce / eliminate the hearing percept.

In view of the above, it can be seen that embodiments include an implantable prosthesis that is configured to actuate to purposely trigger a stapedius reflex in a repeatable manner to at least partially mitigate the effects of the magnetic field induced signal.

In another embodiment, there is an action of operating the prosthesis causes a stapes in the recipient to move in a rocking motion around a long and/or short axes of a footplate thereof, thus at least partially reducing a hearing percept that would otherwise be evoked via the exposure of the MRI field to the prosthesis. Corollary to this is that in an exemplary embodiment, the prosthesis is configured to be operated in an MRI mode where the prosthesis actuates the actuator to cause the above noted movement of the stapes. Note that in some embodiments, normal operation of the actuator to evoke a hearing percept based on normal ambient sounds does not cause the aforementioned rocking, or at least to the extent that such exists, it is at least 50, 60, 70, 80, 90, 91, 92, 93, 94, 95, 96, 97, 98, or 99% less than that which is purposely caused for MRI compatibility functionality.

Embodiments include prostheses configured to be placed into a conditional MRI mode. When in such, the prosthesis can be configured to detect that a voltage has been induced in the leads, and then take action to reduce / eliminate / avoid the hearing percept. In the absence of detection of the voltages when in the MRI mode, the prosthesis will function normally and/or will not actuate at all. In some embodiments, it is only when the voltage is detected that the prosthesis takes action to avoid the resulting hearing percept.

The entrance into the MRI mode can be manually initiated or can be automatically initiated. With respect to the former, a code or a signal can be provided to the implant to engage MRI mode. With respect to the latter, the implant can detect the magnetic field and/or detect the voltage in the leads, and determine that it should enter the MRI compatibility mode.

It is noted that any disclosure of a device and/or system herein corresponds to a disclosure of a method of utilizing such device and/or system. It is further noted that any disclosure of a device and/or system herein corresponds to a disclosure of a method of manufacturing such device and/or system. It is further noted that any disclosure of a method action detailed herein corresponds to a disclosure of a device and/or system for executing that method action / a device and/or system having such functionality corresponding to the method action. It is also noted that any disclosure of a functionality of a device herein corresponds to a method including a method action corresponding to such functionality. Also, any disclosure of any manufacturing methods detailed herein corresponds to a disclosure of a device and/or system resulting from such manufacturing methods and/or a disclosure of a method of utilizing the resulting device and/or system.

Unless otherwise specified or otherwise not enabled by the art, any one or more teachings detailed herein with respect to one embodiment can be combined with one or more teachings of any other teaching detailed herein with respect to other embodiments. Corollary to this is that unless otherwise specified or otherwise not enabled by the art, any one or more teachings detailed herein can be explicitly excluded from combination with one or more other teachings of another teaching detailed herein with respect to other embodiments. In an exemplary embodiment, the prosthesis to which the teachings herein are directed is not a cochlear implant.

While various embodiments have been described above, it should be understood that they have been presented by way of example only, and not limitation. It will be apparent to persons skilled in the relevant art that various changes in form and detail can be made. Thus, the scope of the present invention is defined by the claims.

## Claims

1. An apparatus, comprising:
an implantable portion (244A) of a hearing prosthesis (100, 200A)
wherein the apparatus is configured to at least partially cancel a signal in the implantable portion, the signal resulting from an external magnetic field generated external to a recipient of the hearing prosthesis, wherein:
the signal is a signal generated by the external magnetic field interacting with an electrical
lead (328, 629)
extending between a stimulation output device of the prosthesis and a stimulator and/or receiver of the implantable portion, and
the implantable portion of the hearing prosthesis includes an actuator and electrical leads extending from the actuator (340);
whereby the apparatus is further configured such that the actuator will actuate due to at least some varying magnetic fields in an overall magnetic field that has a value that is greater than at least 1.5T as a result of interaction of the magnetic field with the leads; and/or
the actuator will actuate due to at least some varying magnetic fields having a gradient field value of 20 to 50 mT as a result of interaction of that magnetic field with the leads, wherein the prosthesis is configured to dampen stimulation that results in the hearing percept via functioning of the prosthesis in an MRI mode.

2. The apparatus of claim 1, wherein:
the implantable portion of the hearing prosthesis includes circuitry configured to at least partially cancel the signal;
the implantable portion of the hearing prosthesis is configured with a feedforward and/or feedback circuit that enables the signal to be provided to the circuitry; and
the circuitry is configured to at least partially cancel the signal using the output of the feed forward and/or feedback circuit.

3. The apparatus of claim 1, wherein:
the implantable portion of the hearing prosthesis is configured to provide a cancellation signal that at least partially cancels the signal resulting from the external magnetic field, and/or
the implantable portion of the hearing prosthesis is configured to evoke a hearing percept based on ambient sound while at least partially cancelling the signal resulting from an external magnetic field, and/or
the signal resulting from the external magnetic field is an alternating current that has component(s) having a frequency less than 10kHz and is less than 100 mV, and/or
the hearing prosthesis is a partially implantable hearing prosthesis such that the implantable portion configured such that a cancellation signal is present when exposed to the external magnetic field that at least partially cancels the signal resulting from the external magnetic field.

4. The apparatus of claim 1, wherein:
the prosthesis includes an actuator that is configured to actuate to evoke a hearing percept; and
the prosthesis is configured to generate a low frequency, high amplitude signal so as to reduce and/or eliminate the hearing percept that would be evoked by the hearing prosthesis when subjected to an external magnetic field generated external to a recipient of the hearing prosthesis, which signal actuates the actuator of the prosthesis or
the prosthesis is configured to generate a high frequency, high amplitude signal so as to reduce and/or eliminate the hearing percept that would be evoked by the hearing prosthesis when subjected to an external magnetic field generated external to a recipient of the hearing prosthesis, which signal actuates an actuator of the prosthesis.

5. The apparatus of claim 1, wherein:
the prosthesis is configured to mask an MRI induced voltage to reduce and/or eliminate the hearing percept.

6. The apparatus of claim 1, wherein:
the prosthesis includes an actuator; and
the prosthesis applies a current to the actuator to urge a moving part of the actuator to and/or towards a given location to reduce and/or eliminate the hearing percept that would be evoked by the hearing prosthesis when subjected to an external magnetic field generated external to a recipient of the hearing prosthesis.

7. The apparatus of claim 1, wherein:
the prosthesis is configured to saturate a middle ear of the recipient when functioning to reduce and/or eliminate the hearing percept that would be evoked by the hearing prosthesis when subjected to an external magnetic field generated external to a recipient of the hearing prosthesis.

8. The apparatus of claim 1, wherein:
the prosthesis is a middle ear implant, and wherein the prosthesis is configured to
operate to accommodate the magnetic field gradient of an MRI field with respect to the influence thereof on the prosthesis, thereby at least partially mitigating the effects of the magnetic field induced signal.

9. The apparatus of claim 1, wherein:
the provided signal is a signal that results in a frequency of actuation of the actuator that is at least one of above 7 kHz or below 500 Hz.

10. The apparatus of claim 1, wherein:
the provided signal is a signal that is at least 0.5 V; and
the actuator is configured to operate to evoke a hearing percept having a maximum amplitude at no more than 2 V.

11. The apparatus of claim 1, wherein:
the implantable prosthesis is configured to provide an alternating signal to the actuator and enable voltages induced via the magnetic field to be superimposed onto the alternating signal, thereby at least partially mitigating the effects of the magnetic field induced signal.

12. The apparatus of claim 1, wherein:
the implantable prosthesis is configured to dampen the magnetic field induced signal in the absence of external stimulus.

13. The apparatus of claim 1, wherein:
the implantable prosthesis is configured to actuate to purposely trigger a stapedius reflex in a repeatable manner to at least partially mitigate the effects of the magnetic field induced signal.

## Patentansprüche

1. Vorrichtung, umfassend:
einen implantierbaren Teil (244A) einer Hörprothese (100, 200A), wobei die Vorrichtung so konfiguriert ist, dass sie ein Signal in dem implantierbaren Teil zumindest teilweise aufhebt, wobei das Signal aus einem externen Magnetfeld resultiert, das außerhalb eines Empfängers der Hörprothese erzeugt wird,
wobei
das Signal ein Signal ist, das durch das externe Magnetfeld erzeugt wird, das mit einer elektrischen Leitung (328, 629) in Wechselwirkung tritt, die sich zwischen einer Stimulationsausgabevorrichtung der Prothese und einem Stimulator und/oder Empfänger des implantierbaren Teil erstreckt, und
der implantierbare Teil der Hörprothese einen Aktuator und elektrische Leitungen umfasst, die sich vom Aktuator (340) erstrecken, wobei die Vorrichtung ferner so konfiguriert ist, dass:
der Aktuator aufgrund mindestens einiger variierender Magnetfelder in einem Gesamtmagnetfeld, das einen Wert von mehr als mindestens 1,5 T aufweist, als Ergebnis der Wechselwirkung des Magnetfelds mit den Leitungen betätigt wird; und/oder
der Aktuator aufgrund mindestens einiger variierender Magnetfelder mit einem Gradientenfeldwert von 20 bis 50 mT als Ergebnis der Wechselwirkung dieses Magnetfelds mit den Leitungen betätigt wird,
wobei die Prothese so konfiguriert ist, dass sie die Stimulation dämpft, die zu der Hörwahrnehmung führt, indem die Prothese in einem MRT-Modus arbeitet.

2. Vorrichtung nach Anspruch 1, wobei:
der implantierbare Teil der Hörprothese eine Schaltung umfasst, die so konfiguriert ist, dass sie das Signal zumindest teilweise aufhebt;
der implantierbare Teil der Hörprothese mit einer Vorwärts- und/oder Rückkopplungsschaltung konfiguriert ist, die es ermöglicht, das Signal an die Schaltung zu liefern; und
die Schaltung so konfiguriert ist, dass sie das Signal unter Verwendung des Ausgangs der Vorwärts- und/oder Rückkopplungsschaltung zumindest teilweise auslöscht.

3. Vorrichtung nach Anspruch 1, wobei:
der implantierbare Teil der Hörprothese so konfiguriert ist, dass er ein Auslöschsignal bereitstellt, das das aus dem externen Magnetfeld resultierende Signal zumindest teilweise auslöscht, und/oder
der implantierbare Teil der Hörprothese so konfiguriert ist, dass er eine Hörwahrnehmung auf der Grundlage von Umgebungsgeräuschen hervorruft, während er das aus einem externen Magnetfeld resultierende Signal zumindest teilweise auslöscht, und/oder
das aus dem externen Magnetfeld resultierende Signal ein Wechselstrom ist, der eine oder mehrere Komponenten mit einer Frequenz von weniger als 10 kHz und weniger als 100 mV aufweist, und/oder
die Hörprothese eine teilweise implantierbare Hörprothese ist, sodass der implantierbare Teil so konfiguriert ist, dass ein Auslöschsignal vorhanden ist, wenn er dem externen Magnetfeld ausgesetzt ist, das das aus dem externen Magnetfeld resultierende Signal zumindest teilweise auslöscht.

4. Vorrichtung nach Anspruch 1, wobei:
die Prothese einen Aktuator umfasst, der so konfiguriert ist, dass er betätigt wird, um eine Hörwahrnehmung hervorzurufen; und
die Prothese so konfiguriert ist, dass sie ein Signal mit niedriger Frequenz und hoher Amplitude erzeugt, um die Hörwahrnehmung zu reduzieren und/oder zu eliminieren, die durch die Hörprothese hervorgerufen würde, wenn sie einem externen Magnetfeld ausgesetzt wäre, das außerhalb des Empfängers der Hörprothese erzeugt wird, wobei dieses Signal den Aktuator der Prothese betätigt, oder
die Prothese so konfiguriert ist, dass sie ein Signal mit hoher Frequenz und hoher Amplitude erzeugt, um die Hörwahrnehmung zu reduzieren und/oder zu eliminieren, die durch die Hörprothese hervorgerufen würde, wenn sie einem externen Magnetfeld ausgesetzt wäre, das außerhalb des Empfängers der Hörprothese erzeugt wird, wobei dieses Signal einen Aktuator der Prothese betätigt.

5. Vorrichtung nach Anspruch 1, wobei:
die Prothese so konfiguriert ist, dass sie eine durch MRT induzierte Spannung maskiert, um die Hörwahrnehmung zu reduzieren und/oder zu eliminieren.

6. Vorrichtung nach Anspruch 1, wobei:
die Prothese einen Aktuator umfasst; und
die Prothese einen Strom an den Aktuator anlegt, um einen beweglichen Teil des Aktuators an und/oder in Richtung einer bestimmten Stelle zu drücken, um die Hörwahrnehmung zu reduzieren und/oder zu eliminieren, die durch die Hörprothese hervorgerufen würde, wenn sie einem externen Magnetfeld ausgesetzt wäre, das außerhalb eines Empfängers der Hörprothese erzeugt wird.

7. Vorrichtung nach Anspruch 1, wobei:
die Prothese ist so konfiguriert, dass sie das Mittelohr des Empfängers sättigt, wenn sie dazu dient, die Hörwahrnehmung zu reduzieren und/oder zu eliminieren, die durch die Hörprothese hervorgerufen würde, wenn diese einem externen Magnetfeld ausgesetzt wäre, das außerhalb des Empfängers der Hörprothese erzeugt wird.

8. Vorrichtung nach Anspruch 1, wobei:
die Prothese ein Mittelohrimplantat ist und wobei die Prothese konfiguriert ist, so zu arbeiten, dass sie den Magnetfeldgradienten eines MRT-Feldes in Bezug auf dessen Einfluss auf die Prothese ausgleicht, wodurch die Auswirkungen des durch das Magnetfeld induzierten Signals zumindest teilweise gemildert werden.

9. Vorrichtung nach Anspruch 1, wobei:
das bereitgestellte Signal ein Signal ist, das zu einer Betätigungsfrequenz des Aktuators von über 7 kHz und/oder unter 500 Hz führt.

10. Vorrichtung nach Anspruch 1, wobei:
das bereitgestellte Signal ein Signal ist, das mindestens 0,5 V beträgt; und
der Aktuator so konfiguriert ist, dass er so arbeitet, dass er eine Hörwahrnehmung mit einer maximalen Amplitude von nicht mehr als 2 V hervorruft.

11. Vorrichtung nach Anspruch 1, wobei:
die implantierbare Prothese so konfiguriert ist, dass sie ein Wechselstromsignal an den Aktuator liefert und es ermöglicht, dass durch das Magnetfeld induzierte Spannungen dem Wechselstromsignal überlagert werden, wodurch die Auswirkungen des durch das Magnetfeld induzierten Signals zumindest teilweise gemildert werden.

12. Vorrichtung nach Anspruch 1, wobei:
die implantierbare Prothese so konfiguriert ist, dass sie das durch das Magnetfeld induzierte Signal in Abwesenheit eines externen Reizes dämpft.

13. Vorrichtung nach Anspruch 1, wobei:
die implantierbare Prothese so konfiguriert ist, dass sie betätigt wird, um gezielt einen Stapediusreflex auf wiederholbare Weise auszulösen, um die Auswirkungen des durch das Magnetfeld induzierten Signals zumindest teilweise zu mildern.

## Revendications

1. Appareil, comprenant :
une partie implantable (244A) d'une prothèse auditive (100, 200A),
dans lequel l'appareil est configuré pour annuler au moins partiellement un signal dans la partie implantable, le signal résultant d'un champ magnétique externe généré à l'extérieur d'un bénéficiaire de la prothèse auditive, dans lequel :
le signal est un signal généré par le champ magnétique externe interagissant avec un conducteur électrique (328, 629) s'étendant entre un dispositif de sortie de stimulation de la prothèse et un stimulateur et/ou un récepteur de la partie implantable, et
la partie implantable de la prothèse auditive comporte un actionneur et des conducteurs électriques s'étendant à partir de l'actionneur (340) ;
l'appareil étant ainsi en outre configuré de telle sorte que l'actionneur va s'actionner en raison d'au moins certains champs magnétiques variables dans un champ magnétique global qui a une valeur qui est supérieure à au moins 1,5T en résultat de l'interaction du champ magnétique avec les conducteurs ;
et/ou
l'actionneur va s'actionner en raison d'au moins certains champs magnétiques variables ayant une valeur de champ à gradient de 20 à 50 mT résultant de l'interaction de ce champ magnétique avec les conducteurs, dans lequel la prothèse est configurée pour amortir la stimulation qui a pour résultat la perception auditive via le fonctionnement de la prothèse en mode MRI.

2. Appareil selon la revendication 1, dans lequel :
la partie implantable de la prothèse auditive comporte une circuiterie configurée pour annuler au moins partiellement le signal ;
la partie implantable de la prothèse auditive est configurée avec un circuit de réaction positive et/ou de rétroaction qui permet au signal d'être fourni à la circuiterie ; et
la circuiterie est configurée pour annuler au moins partiellement le signal en utilisant la sortie du circuit de réaction positive et/ou de rétroaction.

3. Appareil selon la revendication 1, dans lequel :
la partie implantable de la prothèse auditive est configurée pour fournir un signal d'annulation qui annule au moins partiellement le signal résultant du champ magnétique externe, et/ou
la partie implantable de la prothèse auditive est configurée pour évoquer une perception auditive basée sur un son ambiant tout en annulant au moins partiellement le signal résultant d'un champ magnétique externe, et/ou
le signal résultant du champ magnétique externe est un courant alternatif qui a une ou des composantes ayant une fréquence inférieure à 10 kHz et est inférieure à 100 mV, et/ou
la prothèse auditive est une prothèse auditive partiellement implantable de sorte que la partie implantable est configurée de manière à ce qu'un signal d'annulation est présent lorsqu'elle est exposée au champ magnétique externe qui annule au moins partiellement le signal résultant du champ magnétique externe.

4. Appareil selon la revendication 1, dans lequel :
la prothèse comporte un actionneur qui est configuré pour s'actionner afin d'évoquer une perception auditive ; et
la prothèse est configurée pour générer un signal à basse fréquence et amplitude élevée afin de réduire et/ou d'éliminer la perception auditive qui serait évoquée par la prothèse auditive lorsqu'elle est soumise à un champ magnétique externe généré à l'extérieur d'un bénéficiaire de la prothèse auditive, lequel signal actionne l'actionneur de la prothèse ou
la prothèse est configurée pour générer un signal à haute fréquence et amplitude élevée afin de réduire et/ou d'éliminer la perception auditive qui serait évoquée par la prothèse auditive lorsqu'elle est soumise à un champ magnétique externe généré à l'extérieur d'un bénéficiaire de la prothèse auditive, lequel signale actionne un actionneur de la prothèse.

5. Appareil selon la revendication 1, dans lequel :
la prothèse est configurée pour masquer une tension induite par MRI afin de réduire et/ou d'éliminer la perception auditive.

6. Appareil selon la revendication 1, dans lequel :
la prothèse comporte un actionneur ; et
la prothèse applique un courant à l'actionneur pour pousser une partie mobile de l'actionneur jusqu'à et/ou vers un endroit donné afin de réduire et/ou d'éliminer la perception auditive qui serait évoquée par la prothèse auditive lorsqu'elle est soumise à un champ magnétique externe généré à l'extérieur d'un bénéficiaire de la prothèse auditive.

7. Appareil selon la revendication 1, dans lequel :
la prothèse est configurée pour saturer une oreille moyenne du bénéficiaire lors de son fonctionnement, afin de réduire et/ou d'éliminer la perception auditive qui serait évoquée par la prothèse auditive lorsqu'elle est soumise à un champ magnétique externe généré à l'extérieur du bénéficiaire de la prothèse auditive.

8. Appareil selon la revendication 1, dans lequel :
la prothèse est un implant d'oreille moyenne, et dans lequel la prothèse est configurée pour
fonctionner pour accommoder le gradient de champ magnétique d'un champ MRI par rapport à son influence sur la prothèse, atténuant ainsi au moins partiellement les effets du signal induit par champ magnétique.

9. Appareil selon la revendication 1, dans lequel :
le signal fourni est un signal qui engendre une fréquence d'actionnement de l'actionneur qui est au moins une fréquence supérieure à 7 kHz ou inférieure à 500 Hz.

10. Appareil selon la revendication 1, dans lequel :
le signal fourni est un signal qui est d'au moins 0,5 V ; et
l'actionneur est configuré pour fonctionner afin d'évoquer une perception auditive ayant une amplitude maximale de pas plus de 2 V.

11. Appareil selon la revendication 1, dans lequel :
la prothèse implantable est configurée pour fournir un signal alternatif à l'actionneur et permettre la superposition de tensions induites par le champ magnétique sur le signal alternatif, atténuant ainsi au moins partiellement les effets du signal induit par champ magnétique.

12. Appareil selon la revendication 1, dans lequel :
la prothèse implantable est configurée pour amortir le signal induit par champ magnétique en l'absence de stimulus externe.

13. Appareil selon la revendication 1, dans lequel :
la prothèse implantable est configurée pour s'actionner afin de déclencher volontairement un réflexe stapédien d'une manière répétable afin d'atténuer au moins partiellement les effets du signal induit par champ magnétique.
